# EUROPEAN PATENT SPECIFICATION

(11) **EP 2 486 406 B1**
(45) Date of publication and mention of the grant of the patent: **10.04.2019**
(21) Application number: 10776400.3
(22) Date of filing: 23.06.2010
(51) Int. Cl.: G01N 33/50, C12N 5/00

(54) **IDENTIFICATION OF SEX-LINKED PROTEINS**
IDENTIFIZIERUNG VON GESCHLECHTSASSOZIIERTEN PROTEINEN
IDENTIFICATION DE PROTÉINES LIÉES AUX CHROMOSOMES SEXUELS

(30) Priority: 23.06.2009 GB 0910826
(43) Date of publication of application: 15.08.2012
(73) Proprietor: Cattle Logic Ltd, Buckland Dinham Frome Somerset BA11 2QS (GB)
(72) Inventor: CUMMING, Ian, Robert, South Glamorgan CF14 4UJ (GB); BREWIS, Ian, Andrew, South Glamorgan CF14 4UJ (GB); PITTS, Sharon, Amanda, South Glamorgan CF14 4UJ (GB)
(74) Representative: Heaton, Joanne Marie
(86) International application number: PCT/GB2010/051041
(87) International publication number: WO 2010/150013

(56) References cited:
- GB-A- 2 360 360
- YEH YUEH-CHIAO ET AL: "STAGE-DEPENDENT EXPRESSION OF EXTRA-EMBRYONIC TISSUE-SPERMATOGENESIS-HOMEOBOX GENE 1 (ESX1) PROTEIN, A CANDIDATE MARKER FOR X CHROMOSOME-BEARING SPERM", REPRODUCTION, FERTILITY AND DEVELOPMENT, CSIRO, EAST MELBOURNE, AU, vol. 17, no. 4, 1 January 2005 (2005-01-01), pages 447-455, XP008073854, ISSN: 1031-3613, DOI: DOI:10.1071/RD04077
- HEID HANS ET AL: "Novel actin-related proteins Arp-T1 and Arp-T2 as components of the cytoskeletal calyx of the mammalian sperm head.", EXPERIMENTAL CELL RESEARCH 1 OCT 2002 LNKD- PUBMED:12243744, vol. 279, no. 2, 1 October 2002 (2002-10-01), pages 177-187, XP002617109, ISSN: 0014-4827
- PARATI K ET AL: "Sex ratio determination in bovine semen: A new approach by quantitative real time PCR", THERIOGENOLOGY, LOS ALTOS, CA, US, vol. 66, no. 9, 1 December 2006 (2006-12-01), pages 2202-2209, XP025076727, ISSN: 0093-691X, DOI: DOI:10.1016/J.THERIOGENOLOGY.2006.07.007 [retrieved on 2006-12-01]
- DATABASE UniProt [Online] 7 February 2006 (2006-02-07), "SubName: Full=Glycerol kinase;", XP002617110, retrieved from EBI accession no. UNIPROT:Q2NKZ8 Database accession no. Q2NKZ8
- DEAN MATTHEW D ET AL: "Adaptive evolution of proteins secreted during sperm maturation: an analysis of the mouse epididymal transcriptome.", MOLECULAR BIOLOGY AND EVOLUTION FEB 2008 LNKD- PUBMED:18056076, vol. 25, no. 2, February 2008 (2008-02), pages 383-392, XP002617111, ISSN: 1537-1719
- JOHNSON L A ET AL: "SEX PRESELECTION: HIGH-SPEED FLOW CYTOMETRIC SORTING OF X AND Y SPERM FOR MAXIMUM EFFICIENCY", THERIOGENOLOGY, LOS ALTOS, CA, US, vol. 52, no. 8, 1 January 1999 (1999-01-01), pages 1323-1341, XP001023997, ISSN: 0093-691X, DOI: 10.1016/S0093-691X(99)00220-4

## Description

This invention relates to methods for the identification of sex-linked proteins, to uses of these proteins, to the proteins thus identified and to cells bearing the proteins. More particularly, the present invention relates to a method for identifying sex-linked proteins in sperm cells and to sperm cells separated according to sex.

In the description which follows the term "sex-linked" is intended to define a protein, polypeptide or gene found on or associated with only one of the X- or Y-chromosomes or to sperm cells carrying an X- or a Y-chromosome.

Mankind has been interested in sex selection for many years. As long ago as 500 B.C., Greek philosophers described methods presumed to have achieved pre-selection of the sex of offspring (reviewed by Betteridge, 1984).

Methods of and prospects for the separation of mammalian sperm according to sex chromosome content ("semen sexing"), have previously been reviewed by others (For example, Amman and Seidel 1982; Gledhill 1983, 1988; Amman 1989; Zarutskie et al. 1989; McEvoy 1992; Cran 1992, 1993; Windsor et al. 1993, and Johnson 1994).

Attempts to separate spermatozoa into X- and Y-chromosome bearing fractions have been based on exploiting differences (proven and unproven) between each subpopulation. These differences include, but are not limited to, mass, motility, size, surface antigenic determinants, DNA content, surface differences and surface charge.

In modern livestock breeding it would be a huge commercial and animal welfare advantage to be able to produce stock of pre-determined sex. For example, in the pig industry there is an increasing resistance to the routine castration of male piglets because of welfare concerns surrounding such castration. Indeed, this practice is no longer allowed in the UK and the phasing out is likely to follow in other countries. If pig farmers could be given the opportunity to produce higher numbers of female pigs for slaughter, the necessity for castration would be reduced.

In the dairy cattle industry, farmers need to produce female herd replacements to ensure that herd numbers are maintained to maximise milk production. A 50:50 male: female sex ratio at birth means that the farmer may produce a male dairy calf for every female replacement that is born. Male dairy calves are often an unwanted by-product of the dairy industry as their only use is for rearing for meat production, and dairy breed muscle conformation is far less suited to this than beef breed conformation. As such male dairy calves often have a very low value in the market place and are often slaughtered at birth - a welfare problem in its own right. A reliable X-bearing sperm enrichment methodology would enable farmers to use less of their herd for producing female replacements (by reducing the chance of producing a male calf) and more of their herd for producing beef / dairy cross animals for superior meat production.

For the beef farmer, a reliable sperm enrichment methodology for Y-bearing sperm would enable them to produce more males for beef production thus improving the total volume of meat sales from the herd (males are 20% heavier than females).

Hence, these breeding issues could be overcome or the associated welfare concerns addressed if a reliable control over the sex of offspring were available such that the semen used for Artificial Insemination was wholly single sex or at least enriched for the preferred sex. In the description which follows, the term "enriched" is intended to describe a population of sperm cells in which the expected 50:50 ratio of nature is skewed to produce a bias in favour of the presence of greater numbers of sperm cells bearing the preferred sex chromosome. Additionally, if the sorted sperm are used in conjunction with a human IVF programme, it will be possible to reduce the incidence of sex-linked diseases such as haemophilia or muscular dystrophy by guaranteeing that only daughters, who generally will be carriers rather than sufferers, are produced as a result of *in vitro* fertilisation.

The functional development of the sperm plasma membrane is described by Holt (1982). Vierula and Rajaniemi (1981) have demonstrated the presence of an uneven distribution of surface proteins in bull spermatozoa. Parks et al. (1987) described the lipid composition of the bovine sperm plasma membrane.

A bovine glycerol kinase is described in the UniProt database at accession no. Q2NK28.

Yeh et al (2005) describe the presence of proteins which are X- or Y-chromosome specific inside sperm cells but concludes that the proteins cannot be used for cell separation.

Heid et al (2002) describe the presence of a contractile actin-related protein in the calyx of the mammalian sperm head, but this is not chromosome specific.

Separation of X- and Y- chromosome bearing cells on the basis of the cells' surface charge is described in GB 2360360.

Parati et al (2006) describe the separation of semen by using real-time PCR.

Dean et al (2008) describe the finding of a group of genes found in the epididymis which are important in reproduction.

The establishment of regional differentiation in the bovine sperm plasma membrane involves the subdivision of the sperm surface into domains which exhibit different antigenic, biophysical and lectin binding properties. Spermatogenic cell antigens appear on the cell surface in sequence as spermatogenesis progresses (Holt 1982).

Blecher et al. (2000), report an approach to the immunological sexing of bovine sperm which is also considered to have commercial potential and is being further developed.

New developments in sperm separation technology (Johnson and Welch 2000), have enabled fluorescence activated cell sorted (FACS) semen samples to be offered commercially.

Conventional sperm sorting is done using Flow Cytometry such as that offered by Sexing Technologies. This approach uses Fluorescence Activated Cell Sorting (FACS) where sperm DNA is stained in all cells in the ejaculate using a fluorescent dye. Each cell is individually sorted and excited with a UV laser. Because the X chromosome is larger than the Y chromosome, X-bearing cells fluoresce more brightly (3.8 times more brightly) than Y-bearing cells. This difference in fluorescence is used as the basis for sorting the cells.

However, this technology is expensive and time consuming to use as each cell has to be individually analysed. A cheaper and / or faster technology that can be applied to semen ejaculates without the need for individual cell analysis is required if the benefits of sex-selection are going to be made available to the livestock industry on a volume basis.

WO/2008/02808 describes an invention which provides an aptamer or pool of aptamers which bind(s) to a target molecule on the surface of a mammalian sperm cell and a method for producing the aptamers.

However, none of the prior art relating to the structure and function of the surface of mammalian sperm cells or any X- and/or Y-chromosome bearing sperm enrichment technique has described the exact identity of any X- or Y-chromosome linked protein or glycoprotein at or on the cell membrane, especially at or on the cell surface. Until this is done, it will not be possible to construct a low cost, reliable and robust technology for the separation of mammalian sperm samples into X- and Y-enriched populations.

It is therefore an object of this invention to provide a method for separating mammalian sperm samples into X- and Y-enriched populations.

The technical approach described in this patent application identifies the presence of sex-chromosome linked proteins at or on the surface of mammalian sperm cells, that is, proteins produced from genes active on only one of the X or Y chromosomes during spermatogenesis. The identification of these sex- linked proteins at the sperm cell surface produces unique surface specific binding targets for the attachment of molecular binding ligands (including antibodies, aptamers and others), which can be used to agglutinate or otherwise enrich for either X- or Y-sperm cell class.

In a first aspect, the present invention provides a method of identifying sex linked proteins in a mammalian sperm cell by identifying selective expression of X- or Y-chromosome linked proteins at or on the surface of the cell, the method including the steps of:-
i) treating sperm cell samples to remove the accessory proteins and other seminal fluid components;
ii) separating the sperm cell samples into X- and Y-chromosome bearing enriched fractions;
iii) selecting an enriched fraction obtained in step ii) and staining, solubilising and subjecting the selected cells to electrophoresis to identify proteins at or on the cell membrane or the cell surface and comparing the results from the selected fraction to those from other fractions to eliminate common proteins,
iv) subjecting the proteins identified in step iii) to mass spectrometry and peptide sequencing, and
v) mapping the sequences from step iv) to recognised databases.

It is preferred that the sperm cells are from livestock such as pigs, cattle, sheep, goats or other farm or domesticated animals and the like, from pet, companion or domestic animals such as cats and dogs, from commercially important animals such as horses especially race horses, and from endangered species in zoos or other conservation and breeding programmes, such as tigers, pandas and apes. Most preferably, and in the examples which follow, the sperm cells are from pigs or cattle. Sperm from poultry may be used, but in poultry, like other birds, the Sex chromosomes are Z and W and it is the male bird which is homozygous. The sperm cells are pre-treated to remove extra-cellular debris on the outer surface of the cell and so reveal only those proteins which are part of the cell and are not part of the accessory proteins or the additional secretions normally present in semen. That is, it is preferred that all extra-cellular material which is not an intrinsic part of the cell membrane, particularly material which is added to or picked up by the cell after it has been formed is removed prior to separation. Hence, is intended to remove the accessory proteins, sugars or other physiological material added to the cells during spermatogenesis, together with any other added secretions or additives, including micro-organisms (especially pathogens such as the HIV virus in the case of human IVF procedures) acquired during the processes of cell membrane assembly, cellular maturation, cell storage, cell surface coating, cellular secretions or any other component of the ejaculate other than the sperm cell.

This step allows for the detection of proteins which are specific to the cell *per se* and not to a protein which may be ubiquitously found in the seminal fluid. The cells may also be capacitated with a capacitation medium such as that described in GB 2360360 B, or with another capacitation medium, again, this is considered to ensure that it is proteins expressed by the cells, especially those found in, on or at the cell membrane which are used for detection. In the methods of the prior art, the washing steps used to clean the sperm cells may not be sufficient to remove the accessory or other proteins present in the seminal fluid and intended to protect the sperm cells from the hostile environment of the female reproductive tract. This can lead to the detection of proteins thought to be associated with the X- or Y-chromosome when they are actually part of the seminal fluid, possibly adhering to the cells surface. In the present application, the term capacitance is intended to refer to changed physiological states of the sperm cell surface in a way that allows it to bind to the oocyte within the oviduct of the female reproduction tract. Capacitation is achieved by removing the coating of accessory proteins which are applied to the sperm surface during sperm maturation in the testes, and which protect sperm from surface damage during their passage through the female reproductive tract.

In the description which follows the present invention will be described with reference to its preferred application which is in the sorting of mammalian sperm cells, particularly sperm cells from pigs or cattle as samples of these sperm cells are readily available, but it is not intended at present that the invention be limited to this application since the method of sperm cell separation employed finds equal utility in other animals.

It is preferred that the cells in step ii) are separated by Free-Flow Electrophoresis. Free-flow electrophoresis (FFE) is a technique where the mixture of particles (cells in the present invention) is introduced in a fine stream into a solution which is flowing perpendicular to the lines of force in an electric field. Electronically charged particles are deflected from the direction of flow at an angle determined by a combination of the flow velocity and the electrophoretic mobility of the particle (cell). The electric field is defined as the region around the electrical charge in which an electric force is exerted on another charge. In FFE particles (cells) flow from the bottom to the top of the chamber where they are collected as fractions. The selected cell fraction used in step iii) may be selected from the extreme poles, the outlying poles or from the midpoint according to the expression level and type of protein desired. Using the method of the invention it is possible to isolate sub-populations of cells which display a different pattern of protein expression. These sub-populations may express major cell proteins at a minimum or reduced level, may over-express minor cell proteins, or may even express different and unusual cell proteins. Generally, as rarely-expressed or minor proteins are sought, the fraction will be selected from an outlying fraction at or close to one or both extreme poles of cell separation.

Preferably, the cells used in step iii) are an X-chromosome bearing cell enriched fraction. Hence, the proteins identified in step iii) are X-chromosome specific proteins or proteins whose expression is regulated by the X-chromosome.

As mentioned above, the proteins or peptides identified are preferably expressed at or on the cell membrane such that they are detectable from outside the cell, for example an epitope, hapten or simply a surface protein. However, proteins or peptides within the cell may also be detected if the moiety, for example an aptamer, affibody or simply a dye used for detection is able to enter the cell.

In a preferred embodiment, the electrophoresis of step iii) is a two-dimensional gel electrophoresis, for example Sodium Dodecyl Sulphate Polyacrylamide Gel Electrophoresis (SDS-PAGE). More preferably, the two-dimensional gel electrophoresis is conducted using strips having an immobilised pH gradient (IPG strips).

Optionally, before the electrophoresis of step iii) the sperm cells may be treated with dithiothreitol (DTT) to disrupt the DNA therein.

The final gel is preferably stained to identify spots of protein. Staining may take place with known protein stains such as Coomassie Blue or silver staining.

In a second aspect, the present invention subjects the sample to nano-liquid chromatography in step iii) to produce fractionated protein samples which are subsequently identified by labelling and mass spectrometry followed by genome mapping.

In either aspect, the sperm cells may be lysed prior to the chromatography.

The fractionated protein samples are preferably labelled prior to mass spectrometry. For example, the samples may be labelled using the isobaric tagging method known as iTRAQ which is based on the covalent labelling of N-terminus and side-chain amines of peptides from protein digestions with tags of varying mass. Digested iTRAQ labelled samples are pooled and analysed by mass spectroscopy and the like. A database search may then be performed using fragmentation data to identify the labelled peptides and hence the corresponding proteins. The fragmentation of the attached iTRAQ tag generates a low molecular weight mass reporter ion that can be used to relatively quantify the peptides and the proteins from which they originated.

The present invention also provides proteins identified by the method for use in the determination of the presence of an X-chromosome in a subject sperm cell or a population of subject sperm cells. Naturally, the presence of an X-linked protein in a cell determines that the cell carries an X-chromosome.

The present inventors have found that the proteins identified herein as protein 1 (SEQ ID NO: 1) or protein 2 (SEQ ID NO: 2) are exclusively produced by the X-chromosome bearing sperm cells. In this respect the problem may be expressed by or regulated by the X-chromosome.

Accordingly, the present invention provides for the use of a protein having the sequence listed in SEQ ID NO: 1 or SEQ ID NO: 2 in the identification of sperm cells bearing an X-chromosome.

The present invention further extends to the use of antibodies raised against the proteins of SEQ ID NO: 1 or SEQ ID NO: 2 and to other ligands binding thereto in the detection of the presence of said proteins, and hence the presence (or absence) of an X-chromosome in or on a sperm cell.

The proteins of SEQ ID NO: 1 or SEQ ID NO: 2 antibodies raised thereto or other ligands able to bind to the protein are usable in a method of determining the presence or absence of an X-chromosome in a sperm cell.

Hence, the present invention also provides a method of determining the presence or absence of an X-chromosome in a sperm cell by determining the presence or absence of protein 1 (SEQ ID NO: 1) or protein 2 (SEQ ID NO: 2) in a sperm cell.

Preferably, the presence or absence of an X-chromosome in a sperm cell is determined by the binding of an antibody raised against protein 1 (SEQ ID NO: 1) or protein 2 (SEQ ID NO: 2) to the sperm cell.

Alternatively, since proteins 1 (SEQ ID NO: 1) and 2 (SEQ ID NO:2) are accessible from the extra-cellular environment and so may be at the cell membrane, on either the inner (intracellular) or outer (extracellular) side of the membrane, the presence or absence of an X-chromosome in a sperm cell may be determined by fluorimetric or colorimetric or other staining methods, which may be detected visually, since the uptake of ligand is greater in X-bearing cells than in Y-bearing cells. The resulting difference in appearance, for example, fluorescence, is much greater than the 3.8% currently associated with conventional FACS and described above and hence may be more readily detected. The staining or other colorimetric or fluorescent method may use antibodies, affibodies, aptamers or the like.

Without wishing to be bound by theory, the present inventors believe that it is unlikely that Y-chromosome bearing sperm cells will express an X-chromosome specific protein to which the ligand binds and thus the Y-bearing sperm cells should show a reduced reaction to the ligand. However, under rare biological conditions Y-bearing cells could express an X-specific protein at the cell surface but such an event will be much less common than an X-specific protein being expressed in greater quantities on X-bearing cells. At the selection stage of the method any protein which shows an unacceptable binding to a Y-chromosome bearing sperm may be eliminated as a candidate selective protein.

The use of an aptamer or affibody, in place of an antibody, provides an ability to penetrate the surface membrane of an intact live cell - which hasn't previously been possible. Hence, these peptide ligands can carry a colorimetric moiety into a cell.

Embodiments of the invention will now be described, purely by way of example, with reference to and as illustrated by Figures 1 to 10 of the appended drawings, of which
Figure 1 is a photograph showing the results of two-dimensional gel electrophoresis of whole sperm cells from cattle and pigs and showing the gel position of X-chromosome linked protein 1 (pig sperm);
Figure 2 is a photograph showing the results of two-dimensional gel electrophoresis of whole sperm cells from cattle and pigs and showing the gel position of X-chromosome linked protein 2 (cattle sperm);
Figure 3 is a photograph showing MicroPharm antibody from sheep 0991 binding to Protein 1 at the surface of porcine sperm in two different binding patterns (Figures 3a and b 0991) and a sheep control (Figures 3a and b Sheep Control) where Figures 3a are images taken with fluorescence and phase contrast and Figures 3b are images taken with fluorescence only;
Figure 4 shows the identity of X-chromosome linked protein 1 (SEQ ID NO: 1) and its sequence homology to a MASCOT identified actin-related protein;
Figure 5 is a photograph showing commercial antibody (Com 2) binding to Protein 2 at the surface of porcine sperm. The Com 2 antibody binds to the apical ridge of sperm with some staining of the equatorial subsegment whereas the Mouse Control, where no Com 2 antibody is used does not show the apical ridge binding. The control slide shows no binding of Com 2 to Protein 2 at the surface of porcine sperm;
Figure 6 is a series of photographs showing MicroPharm antibody binding to Protein 1 at the apical ridge and equatorial subsegment of cattle sperm, and mouse antibody control, using phase contrast and fluorescence microscopy;
Figure 7 shows the identity of X-chromosome linked protein 2(SEQ ID NO: 2) and its sequence homology to a MASCOT identified glycerol kinase protein;
Figure 8 is a photograph showing commercial antibody binding to Protein 2 (Com 2) showing binding to the apical ridge and some staining of the equatorial subsegment at the surface of cattle sperm, and showing a control;
Figure 9 shows the amino acid sequences of Protein 1 (SEQ ID NO: 1) and Protein 2 (SEQ ID NO: 2) respectively;
Figure 10 is a trace of FACS X- and FACS Y-enriched sperm samples incubated in fluorescent antibodies to Proteins 1 (Figure 10a) and 2 (Figure 10b) and then cell sorted to measure levels of fluorescence in each population;
Figure 11 is a pair of graphs showing the results of a porcine FACS analysis profiles for unseparated cells stained with antibodies to Protein 1 (Figure 11a) and Protein 2 (Figure 11b);
Figure 12 is a graph showing the results of FACS enriched bovine sperm samples showing X-enrichment at low fluorescence with antibody to Protein 1;
Figure 13 is a graph showing the results of FACS enriched bovine sperm samples showing X-enrichment at low fluorescence with antibody to Protein 2, and
Figure 14 is a diagram of spermatogenesis illustrating preferential expression of X-linked proteins by X-bearing cells.

### Example 1: preparation of sperm samples for two dimensional gel electrophoresis (2DGE)

### Sperm Preparation and antibody binding were carried out as follows:-

Sperm cells were incubated overnight in TEST-Yolk ((N-Tris[hydroxymethyl]methyl-2-aminoethane-sulphonic acid)-Tris yolk) buffer (Ijaz and Hunter 1989) to remove extra-cellular material adhering to and to capacitate the cells. After incubation the cells were centrifuged at 1,000g (-2,800 rpm) for 30 minutes at 5°C after spinning, the supernatant was poured off and the cells were resuspended in 10ml of the separation buffer before being transferred into a 15ml tube to be recentrifuged at 1,000g (2,500rpm for 15ml tubes) for 10 minutes at 5°C.

After centrifugation, the supernatant was removed and the cells were resuspended in a further 5ml of the separation buffer. This suspension was then centrifuged once more at 1,000g (2,500rpm for 15ml tubes) for 10 minutes at 5°C before the supernatant was removed and the cells were resuspended in 1ml of the separation buffer.
The resulting sperm cell samples are either:
a) subjected to surface staining of whole cells and then solubilised and subjected to 2DGE as follows or;
b) first solubilised, then stained and subjected to 2DGE as follows:
   Immobilised pH gradient (IPG) strips are rehydrated (overnight) in a rehydration/lysis Buffer prepared by making up urea and thiourea stock solutions, allowing time to fully dissolve. A 5ml aliquot of the rehydration/lysis buffer is taken and mixed with CHAPS (3-[(3-Cholamidopropyl) dimethylammonio]-1-propanesulfonate), bromophenol blue and specific IPG buffer components without DTT (Dithiothreitol).

This is the stock solution and can be left on the bench for use for a week. DTT is added just before use. Details of the stock solutions are provided in Table 1 below.

**Table 1**

| Final Conc. | | | Stocks | | |
|---|---|---|---|---|---|
| 7M | Urea | 21.63g | 50ml | 5ml | 2ml |
| 2M | Thiourea | 7.62g | | | |
| 4%(w/v) | CHAPS | | | 0.2g | |
| 0.005%(w/v) | Bromophenol blue | 0.01g in 1ml | 1% | 25µl | |
| 0.5%(v/v) | Specific IPG (immobilised pH gradient) Buffer | | | 25µl | |
| 20mM | DTT | 0.154g in 1ml | 1M | | 40µl |

### Strip Rehydration

Add 450µl Rehydration Buffer to each well to be rehydrated. Put 24cm IPG strips, after removing plastic backing, in wells and place gel face down into the reswelling tray. Add 3-4ml DryStrip Cover Fluid (GE HealthCare) to cover each filled lane and leave to rehydrate overnight.

### Isoelectric Focusing (Overnight)

### Sample Preparation - Surface Labelling

The required number of sperm cells are collected (typically between 5 and 50 million) and centrifuged at 12,000g for 4 minutes in buffer. After centrifugation, as much supernatant as possible is removed. The cells are then resuspended in 50µl of the FFE separation buffer (at a pH of 8.5). 2µl (800pmol) of CyDye working solution is added to the sample which is kept on ice for 30 minutes in the dark. The reaction is stopped by adding 20µl 10mM lysine and leaving for another 10 minutes in the dark. The sperm cells are then centrifuged at 12,000g for 4 minutes. The supernatant is then removed and 50µl of the Lysis buffer prepared as above is added. The cells are then lysed for 30mins with vortexing for 20 seconds every 10 minutes in the dark. The lysed cells are then centrifuged again and the supernatant used in a 2D Clean up kit (GE HealthCare).

### Sample Preparation - Whole Cell Lysate Labelling

The required number of sperm cells are collected (typically between 5 and 50 million) and centrifuged at 12,000g for 4 minutes in buffer. After centrifugation, as much supernatant as possible is removed. The cells are then resuspended in 50µl of DIGE Lysis buffer [Urea (7M), Thiourea (2M (Tris (30mM), CHAPS (4%)] and lysed for 30mins with vortexing for 20 seconds every 10 minutes. The lysed cells are then centrifuged again and the supernatant used in a 2D Clean up kit (GE HealthCare). At the end of 2D Clean up the pellet is resuspended in 10µl DIGE lysis buffer. 1µl of CyDye working solution (400pmol) is added to the solution and left in the dark for 30 minutes. Next 1µl of 10mM lysine is added and left to stop the reaction for 10 minutes in the dark.

### Anodic Cup Loading

It is ensured that the cup loading manifold is level on the IPGphor platform. DryStrip Cover Fluid is poured evenly onto the manifold channels. The IPG strips are carefully removed strips from the reswelling tray, rinsed in distilled water and the edge/s blotted on filter paper. Each strip is placed into the manifold "gel side up" and centred in the wells. Two wicks per strip are separated and distilled water is put onto each. The wicks are placed onto either end of each strip to overlap the end of the strip and the electrode (when applied) at each end. The electrodes are put in place at either end of the strip(s) and the cams swivelled to the closed position. The cups are then placed on top at the anodic end of the strips ensuring that they are fitted securely and are not on centred on protrusions.

After briefly centrifuging the cell samples, the supernatant is carefully loaded into the bottom of the cup. It is noted which sample is applied to each strip. Each strip is checked to ensure that it is totally covered by fluid before closing lid ready for overnight focussing, according to the conditions given in Table 2.

**Table 2**

| IsoelectricFocusingConditions for 24cm 3-10NL Strips | | | |
|---|---|---|---|
| | Voltage (V) | Time | kVh |
| 1 Step and Hold | 500 | 1hr | 0.5 |
| 2 Gradient | 1,000 | 7hr | 5.2 |
| 3 Gradient | 10,000 | 3hr | 16.5 |
| 4 Step and Hold | 10,000 | 4hr | 40 |
| 5 Gradient | 500 | 1hr | 5.2 |
| 6 Step and Hold | 500 | 6hr | 30*** |
| Total | | | (45-60) |

| | | | |
|---|---|---|---|
| *** not necessary but used to keep a small amount of current through strip before next step. | | | |

### Current limit 75µA per strip

### Second Dimension SDS-PAGE (sodium dodecyl sulphate -polyacrylamide gel electrophoresis)

The gel casting cassette is set up to pour gels which are made up by mixing the gel mix omitting APS (ammonium persulphate) and TEMED (Tetramethylethylenediamine). When ready to proceed add fresh APS and TEMED and pour into the filling channel. Immediately spray 0.1% SDS solution carefully to the top of all gels. Wrap the top in cling film and allow to set for at least 5 hours before dismantling the casting cassette.

Prepare buffers as set out in Table 3.

**Table 3**

| Equilibration BufferStock (200ml) | | | | | | | |
|---|---|---|---|---|---|---|---|
| 50mM | | Tris-HCl pH8.8 | | | 6.7ml | | 1.5M Stock |
| 6M | | Urea | | | 72.07g | | |
| 2% (w/v) | | SDS | | | 4.0g | | |
| 30% (v/v) | | Glycerol | | | 69ml (84.2g) | | 87%(v/v) MW 92.09 |
| 0.002% (w/v) | | Bromophenol blue | | | 400µl | | 1% Stock |
| NB: Add ∼100ml water to Urea. When partially dissolved, add the glycerol Once the solution is clear add the SDS. | | | | | | | |

| 10X SDSElectrophoresis Buffer | | | | | | | |
|---|---|---|---|---|---|---|---|
| | | | MW | 5L | 1.5L | 800ml | |
| 250mM | Tris | | 121.14 | 151.25g | 45.4g | 24.2g | |
| 1.92M | Glycine | | 75.07 | 720.5g | 216.2g | 115.28g | |
| 1.0% (w/v) | SDS | | 288.38 | 50g | 15g | 8g | |

Make up 10X SDS buffer, allowing it to be thoroughly mixed before diluting to make the running buffers. Make up 5L Anodic Buffer 1X SDS Electrophoresis Buffer (500ml in 5L) for the Lower Chamber and chill in a cool room or refrigerator. Make up 1L Cathodic Buffer 2X SDS Electrophoresis Buffer (200ml in 1L) for the Upper Chamber and chill in a cool room or fridge.

### Agarose Sealing Solution

Switch on heating block to 100° C. Allow 1 aliquot per gel, melt for 10mins then reduce temperature to 65° C until used. Add 125mg Agarose plus 25ml 1X SDS Electrophoresis Buffer (from 5L Anodic Buffer) and add a few grains of bromophenol blue for colour (or add 50µl 1% stock). Boil in microwave and store at 65° C ready for use. This can be stored in the fridge in aliquots and melted at 100° C before being kept at 65° C

### IPG Strip Equilibration

Equilibrate each IEF strip with 15ml DTT Equilibration Buffer (150mg DTT per 15ml stock - 1% (w/v)) for 10-15 minutes with rocking. Each strip is equilibrated with 15ml IAA Equilibration Buffer (375mg IAA-iodoacetamide per 15ml stock - 2.5% (w/v)) for 10-15 minutes with rocking. Each IPG strip is then removed with forceps and rinsed in 1X SDS (anodic) Electrophoresis buffer before being carefully put into the top of the gel plates, acidic side on the left, and slid down onto the gel using old 7cm strips avoiding the introduction of air bubbles before covering with agarose sealing solution and waiting at least 1 minute for the agarose to cool and solidify before putting the gels in the chamber.

### 2D Electrophoresis

The electrophoresis unit is filled with 4L 1X SDS Electrophoresis Buffer and the pump turned on. The anode assembly is inserted and the cooler switched on and set temperature (10° C or 12° C). the gels are inserted into the unit filling any unused spaces with blank cassette inserts and the anodic buffer added to the lower chamber (down the side) until the level reaches "LBC start fill" line. The upper buffer chamber (UBC) is soaked in a container of anodic buffer before being put on and pushed down over the cassettes. All of the cathode buffer is poured into the UBS and the LBC topped up to ensure that the levels are equal in both chambers. The chambers are closed by a lid and set up for electrophoresis as detailed below.

**Overnight Run 12°C**

| Step | mA/gel | Voltage (V) | W/gel | Time |
|---|---|---|---|---|
| 1 | 10 | 80 | 1 | 1hr |
| 2 | 12 | 150 | 2 | 14-16hrs |

**Day Run 10°C**

| Step | mA/gel | Voltage (V) | W/gel | Time |
|---|---|---|---|---|
| 1 | 10 | 80 | 1 | 1hr |
| 2 | 38 | 500 | 13 | 4.5-6hrs |

The units are run until the bromophenol blue reaches the bottom of the gel. For an overnight run, the next morning switch to STEP 2 day run until dye front reaches bottom of gel.

The completed gels are then scanned by Typhoon imaging and the images transferred to a DeCyder computer for analysis using the DeCyder computer analysis settings protocol for this procedure. After scanning the fluorescent images and uploading them to DeCyder software the gels are then stained by either silver staining or Coomassie staining as below.

### Gel Electrophoresis Silver Staining/ Colloidal Coomassie Staining

Silver staining is conducted as set out below.

**Silver Staining**

| Step | | Solution | Volume/gel | Time |
|---|---|---|---|---|
| 1 | Fix | 25ml acetic acid 10 % (v/v); 100ml ethanol 40 % (v/v) | 250 ml | 30 min |
| 2 | Sensitizer | 75 ml ethanol; 10 ml Na-thiosulphate (5 % (w/v) stock); 17g Na-acetate* Add thiosulphate just before use NO GLUTARALDEHYDE | 250 ml | 30 min |
| 3 | Wash | H2Odist | 250 ml | 5 min |
| 4 | Wash | H2Odist | 250 ml | 5 min |
| 5 | Wash | H2Odist | 250 ml | 5 min |
| 6 | Silver | 25 ml silver nitrate (2.5 % (w/v) stock) NO FORMALDEHYDE | 250 ml | 20 min |
| 7 | Wash | H2Odist | 250 ml | 1 min |
| 8 | Wash | H2Odist | 250 ml | 1 min |
| 9 | Developer | 6.25 g Na-carbonate*; 100 µl formaldehyde (37 % (w/v) stock) | 250 ml | Up to 10 min |
| | | Can choose to change solution after 1 min. | | |
| 10 | Stop | 3.65 g EDTA* | 250 ml | 10 min |
| 11 | Wash | H2Odist | 250 ml | 5 min |
| 12 | Wash | H2Odist | 250 ml | 5 min |
| 13 | Wash | H2Odist | 250 ml | 5 min |
| 14 | Preserve | 5 ml acetic acid 1% (v/v) | 500 ml | - |

| | | | | |
|---|---|---|---|---|
| **) The powders are already preweighed in bags.* | | | | |

If not staining on the same day, remove gel from fix solution and store in 5% acetic acid, 5% ethanol in the fridge until ready for staining. Then start from the beginning again. (12.5ml acetic acid, 12.5ml ethanol, 225ml water)

### Colloidal Coomassie

Follow instructions for Invitrogen colloidal Coomassie staining kit. Scan gel after rinsing in water.

### Scanning

Save the file first by clicking on the folder icon
Settings should be the following:
Transmissive
256 scales grey
600dpi
No Descreen
No Filter
Do a Preview and make the scan size correct for the gel.
Scan and save as a .tif file.

### Example 2: Preparation of sperm samples using nano liquid chromatography (Nano-LC) and mass-spectrometry.

Proteins were identified by subjecting Nano-LC fractions derived from whole sperm cells from pigs and cattle treated as in example 1 to obtain capacitated sperm cells to iTRAQ labelling and mass-spectrometry analysis to establish peptide sequence data. This peptide sequence data was subsequently entered into MASCOT, a powerful search engine that uses mass spectrometry data to identify proteins from primary sequence databases.

Nano-liquid chromatography was performed on mammalian sperm samples using the following laboratory protocols.

### Protocol for Sample Preparation for Nano-LC

The samples received for NanoLC are cell pellets which are subjected to protein extraction followed by protein assay, trypsin digestion to yield a peptide mixture and subsequent iTRAQ labelling.

### Protein Extraction

Protein extraction is performed using one of three methods:-

### (a) 1% NP40 in TEAB:

### Lysis Buffer

To make 10 ml mix
1. 1 ml of NP40 (detergent) (10% v/v stock)
2. 200 µl of Triethylammonium Bicarbonate (TEAB) (1 M stock)
3. 100 µl of PMSF (protease inhibitor) (20 mg/ml stock)
4. Make the volume to 10 ml with Distilled Water

### Protocol

1. Lyse cells in 500 µl lysis buffer for 15 min. on ice
2. Spin at 13,000 RPM for 5 min. at 40C in a refrigerated centrifuge (Allegra 21R, Beckman Coulter) to clarify
3. Remove supernatant & quantify for proteins present
4. Also store pellet at -800C (for use in future, if needed)

### (b) 1% SDS in TEAB:

### Lysis Buffer

To make 10 ml
1. 1 ml of SDS stock (detergent) (10% w/v stock)
2. 200 µl of Triethylammonium Bicarbonate (TEAB) (1 M stock)
3. Make the volume to 10 ml with Distilled Water

### Protocol

1. Lyse cells in 100-500 µl lysis buffer for 30 min. at room temperature
2. Boil sample in 1% SDS for 10 min. (optional)
3. Spin at 13,000 RPM in microfuge for 5 min. at room temperature to clarify
4. Remove supernatant & quantify for proteins present
5. Also store pellet at -800C (for use in future, if needed)

### (c) 2% CHAPS in Urea/Thiourea:

### Lysis Buffer

To make 5 ml
1. 5 ml Urea/Thiourea stock (containing 7 M Urea and 2 M Thiourea)
2. 100 mg of CHAPS added to urea/thiourea stock solution

### Protocol

1. Lyse cells in 100-500 µl lysis buffer for 30 min. at room temperature
2. Spin at 13,000 RPM microfuge for 5 min. at room temperature to clarify
3. Remove supernatant & quantify for proteins present
4. Also store pellet at -800C (for use in future, if needed)

### Protein Assay

The Protein Assay is undertaken using the Bicinchoninic Acid Protein Assay Kit (Sigma). The principle of the bicinchoninic acid (BCA) assay is based on the formation of a Cu2+-protein complex under alkaline conditions, followed by reduction of the Cu2+ to Cu1+ due to the reducing nature of proteins.

The Protein assay is performed in a 96 well plate and a Standard Curve with BSA (1 mg/ml) is used (Scheme as shown in Table 4 below.) according to the following protocol:
1. Serial dilutions of BSA are prepared using 1 mg/ml solution in column 12 using 200 µl of BSA solution in H-12 and 100 µl of water in B-12 to G-12.
2. Add 100 µl of solution from H-12 to G-12 mix and then take 100 µl from G-12 to F-12 and so on until B-12. A-12 is left blank.
3. Take 50 µl each of diluted standard and put it in corresponding wells 1, 2 and 3 of same row (triplicate)
4. Add 25 µl of each sample in duplicate in column 4 and 5
5. BCA working reagent is prepared by mixing 50 parts of reagent A with 1 part of reagent B.
6. Add 200 µl of BCA working reagent in each well, cover with plastic seal and incubate for 30 min. at 370 C
7. Let the colour develop and then take readings on ELISA plate reader at 570 nm.
8. If some sample has high protein conc. and is out of range then dilute it further and repeat.
9. After this a standard curve is obtained plotting Conc. of BSA standard against absorbance at 570 nm.
10. The protein concentration of samples is worked out by taking the average absorbance of the samples in duplicate and estimating it using the standard curve.
11. For urea/thiourea CHAPS extraction, the cell lysate is subjected to 2-D cleanup (method outlined in detail in protocols for running 2-D gels) prior to protein assay. This is because BCA assay can tolerate a maximum of 3 M urea conc.

**Table 4**

| Scheme forPreparationofProteinAssay forNano-LCAnalysisUsinga 96Well Plateas Described in Steps 1 to 11 Above forBothSamples andReagents | | | | | | | | | | | | | | | | | | | | | | |
|---|---|---|---|---|---|---|---|---|---|---|---|---|---|---|---|---|---|---|---|---|---|---|
| | 1 | | 2 | | 3 | | 4 | | 5 | | 6 | 7 | | 8 | | 9 | | 10 | | 11 | | 12 |
| A | | 0.0 | | | | | | | | | | | | | | | | | | | 0.0 | |
| B | | 0.015625 | | | | | | | | | | | | | | | | | | | 0.015625 | |
| C | | 0.03125 | | | | | | | | | | | | | | | | | | | 0.03125 | |
| D | | 0.0625 | | | | | | | | | | | | | | | | | | | 0.0625 | |
| E | | 0.125 | | | | | | | | | | | | | | | | | | | 0.125 | |
| F | | 0.25 | | | | | | | | | | | | | | | | | | | 0.25 | |
| G | | 0.5 | | | | | | | | | | | | | | | | | | | 0.5 | |
| H | | 1 | | | | | | | | | | | | | | | | | | | 1 | |

### BSA mg/ml (50 µl) Sample (duplicate) BSA (mg/ml) Dilution (triplicate) (25 µl) (Serial dilution)

### Trypsin digestion and iTRAQ labelling

Trypsin digestion is done after reducing the sample, denaturing it, and blocking the Cysteines. The process is undertaken using the Applied Biosystems iTRAQ labelling kit and protocol.

### Denaturant (2% SDS).

### Reducing Reagent.

### Cysteine Blocking Reagent.

1. To each of up to four sample tubes containing 5 to 100 µg of sample (or the precipitated pellet from acetone precipitation), add 20 µL Dissolution Buffer.
2. Add 1 µL of the Denaturant in the kit and vortex to mix.
3. To each sample tube, add 2 µL Reducing Reagent.
4. Vortex to mix, then spin.
5. Incubate the tubes at 60°C for 1 hour.
6. Spin to bring the sample to the bottom of the tube.
7. To each tube, add 1 µL Cysteine Blocking Reagent.
8. Vortex to mix, then spin.
9. Incubate the tubes at room temperature for 10 minutes.

### Digesting the Proteins with Trypsin

1. To each sample tube, add 0.8 µg (2 µL) of the trypsin solution from aliquots prepared previously.
2. Vortex to mix, then spin.
3. Incubate the tubes at 37°C overnight (12 to 16 hours).
4. Spin to bring the sample digest to the bottom of the tube.

### iTRAQ™ Labelling

### Reagents : Ethanol. iTRAQ™ Reagents 114-117.

1. Allow each vial of iTRAQ™ Reagent required to reach room temperature.
2. Spin to bring the solution to the bottom of the tube.
3. Add 70 µL of ethanol to each room-temperature iTRAQ™ Reagent vial.
4. Vortex each vial to mix, then spin.
5. Transfer the contents of one iTRAQ™ Reagent vial to one sample tube.
6. Vortex each tube to mix, then spin.
7. Incubate the tubes at room temperature for 1 hour.

### In-solution trypsin digestion (without iTRAQ labelling)

1. Dissolve precipitate after 2-D clean up in 100 µL of 25mM Ammonium Bicarbonate (Ambic)
2. Add 1 µL of 1M of freshly made DTT
3. Incubate at 56°C for 1 hour
4. Cool for 15 minutes
5. Add 11 µL of freshly made 0.5M lodoacetamide
6. Incubate for 1 hour at room temperature
7. Add 0.8 µg (2 µL) of trypsin from stock
8. Incubate at 37°C overnight.
9. Sample can then be dried and resuspended in water with 0.05% TFA for NanoLC

### Protocol for Nano-LC

Pre-run Procedures (machine make and model).

### Initial Checks :

1. Check Pump Wash, beside pumps - at top (50% Isopropanol/Water) and replace with fresh solution if half full
2. Check Transport Solvent, beside needle - In auto sampler (2% ACN [Acetonitrile] in water/0.05% TFA [trifluoroacetic acid]) and replace with fresh solution if half full
3. Check Wash bottle at the bottom on left (50%ACN/water) and replace with fresh solution if half full
4. Check waste, to the right of the machine - replace if needed and store the filled bottle in metal cabinet in ice-machine room until disposal
5. Check computer is on
6. Check Chromeleon Server is on
7. Check Chromeleon Software is on (if not start the software)
8. Check the UV lamp is on (UV lamp takes about 30 minutes to warm up)

### Replenishing of buffers and wash fluids

1. Buffers A and B should be replaced every week (composition described below)

| Buffer A (2% ACN) | | Buffer B (90% ACN) | |
|---|---|---|---|
| ACN | 10ml | ACN | 450ml |
| Water | 490ml | Water | 50ml |
| TFA v/v)* | 250µl (0.05% | TFA* | 250µl (0.05% v/v) |

| | | | |
|---|---|---|---|
| * Use a new TFA vial every month (date labelled on opening) | | | |

2. All wash fluids should also be replaced weekly
3. Make sure to discard all the left over buffers and wash fluids

### Priming of the system

1. In the Control Module (Control.pts) of Chromeleon , select the Home Tab
2. Turn on pumps for normal run:
   Standard conditions:
   Left Valve in 10-1 position
   Right Valve in 1-2 position
   Loading Pump flow rate 25 µl per minute
   Micro Pump flow rate 0.300 µl per minute
3. Wait for pressure to settle.
4. Check the pressure over the column. Pressure should be about 180-200 bar (100 bar for shorter column). Note it for information and diagnostic purposes. If the pressure continues to increase, it might be indicative of a block in the flow-path
5. Allow the system to equilibrate to Buffer A, for at least 20 minutes.
6. Check that there is buffer coming through the system to the needle of the Probot.
7. Turn on Manual Acquisition (Control Module, Status Tab) and check that the A214 is flat and does not change more than 1 or 2 mAU. If More, then system has the remnants of previous runs and need to be washed by running buffers for longer periods.

### Running a sample (Batch mode)

### LHS rotor valve

10-1 Loading pump to pre to waste; gradient to column
1-2 Loading pump to waste; precolumn in line with column

### RHS rotor valve

10-1 SCX on-line
1-2 SCX off-line

To obtain the required data and appropriate spot-sets on MALDI plates using Probot (microfraction collector - Dionex), NanoLC has to run a batch depending upon the type of run. Usually, two types of run are undertaken (i) 1-D Long run, and (ii) Short salt plug run
(i) 1-D Long run - This run is of 210 min. duration and involves only reverse phase column. The spotting on the MALDI plate starts immediately after the start of the run and continues for the duration of the run at 8 sec. interval.
(ii) Short salt plug run - This module involves a series of 6 runs of 75 min. each and also use Bio-SCX cation exchange column along with reverse phase column. The spotting start at 25 min. of each run and proceeds for 34 min. at 8 sec. interval thereby producing six sets of spots.

N.B. You need to turn off the Manual Acquisition before running a Batch otherwise the machine will give an error message.

### Sample preparation:

### Blank

This aborts the inject element of the Programme. These are useful to check the baseline before running a sample, cleaning after running a sample and to monitor the cleanliness of the column.

### Cytochrome C

Aliquots of Cytochrome C are located in the freezer which need to be diluted 1:80 with water + 0.05% (v/v) TFA. For sample runs, we use Cytochrome C as internal control to keep an eye on performance of the system.

### Unknown

Usually made up in 0.05% (v/v) TFA/Water.
Place the Cytochrome C and Unknown in the Autosampler at the positions which match with their description on the program file. Check the positions are correct.

N.B. Batch is aborted if no sample is in the location selected.

### Setting up a batch run

A batch consists of one of more vials. Each vial must have a Programme File and a Quantification File. Only the Programme File is changed. The easiest
way to set up a Programme File is to select a previous one and File.. Save as... into a different folder or with a different name. This should change the Status to "Single" meaning that it has not been run yet.

Each vial has the following parts:

### Name Type Position Inj Vol Program Method Status

Name - Just for information
Type - Use "Blank", "Cyto C" and "Unknown".
Position - Specify position of vials
Inj Vol - Depends on inject type but we are currently using 20□l
Program - The KEY: describes the details of program which include details on the pumps, pressures and gradients
Method - This is the quantitation aspect. Use "Default"
Status - "Single" - not run; "Finished" or "Running" or "Interrupted"

After placing the sample, control and other vials in the auto sampler, appropriate run (1-D long run or Short salt plug) is selected and the file set is saved. Also, the matching spot-set program is selected on micro carrier software for Probot plating after preparation of probot. The plate is aligned and the probot program is started which after doing a series of checks give message that it is waiting for "Start" input from LC.

The saved file set on LC is put to "ready check" which checks and points out errors in the process, if any. After the ready check is successful the run is started and the whole process is automated after this.

### Probot preparation and maintenance

### Making up Matrix

1. Add 1ml 70% ACN/0.1% (v/v) TFA to one the vials purchased from Sigma. These are preweighed at 10mg so will give a 10mg/ml solution.
2. Vortex well.
3. Spin in microfuge - 5 min at full speed.
4. Dilute to 2 mg/ml (suggest take 800µL and add to 3.2ml).
5. Add 2.0 µL of 50pmol/µL u-Fib spike. Glu-fib is obtained as white powder, 1mg of which is dissolved in 636.9 µl of Chromasolv water to get stock solution having 1nmol/ µl.
6. This stock solution is diluted 1:20 in 70% ACN/ 0.1% TFA to get 50 pmol/µl concentration which is used for the experiment.

### To make 70% ACN/0.1%TFA

| | 10ml | 25ml |
|---|---|---|
| ACN | 7ml | 17.5ml |
| H2O | 3ml | 7.5ml |
| TFA | 10µL | 25µL |

Load the matrix into Probot and prime by filling the syringe and pumping the matrix solution at least five times.

### Microcarrier Software

Usually, a presetup Application corresponding to the two types of run is used. The plate MUST be aligned every time when a run is setup. Our normal spotting template allows rows of 52 spots. These can be applied at almost every rate but normally we use between 8 sec and 12 seconds per spot.

### Putting calibration spots (MW standards) to the plate

These would normally be made up in the same matrix as used for spotting the fractions. Usually use Cal Mix 1 and Cal Mix 2. These are kept in 2µl aliquots in the freezer. Add 23 µl of matrix and use 0.5 µl per calibration spot to the plate.

### Flushing out the matrix after the run

This is required to avoid matrix crystallisation within the probot tubings. The vial is washed and loaded with 70% ACN/0.1% (v/v) TFA and the matrix is washed by filling the syringe and pumping the solvent (70% ACN/0.1% (v/v) TFA) at least five times.

At the end of the day or after a run, if going to use the following day, put into wash conditions, say 50% Buffer B

### Wash Conditions

### Loading Pump 5 µl/min

### Micro Pump 0.050 µl/min

IMPORTANTLY ENSURE THAT THERE IS ENOUGH BUFFER SO SYSTEM DOES NOT RUN DRY. Ensure that there is at least 100ml per day of buffer A & B if there is no run taking place. If there is a run taking place ensure there is 300 ml of buffer A & B. If these buffers need changing then turn pumps off. Add buffers and then set pumps to wash conditions. ALSO set Buffer B to 50 %. After replacement of buffer or wash fluids, both the pumps are purged for 10 min.

There is also a "Long Run" Program that can be placed at the end of a batch to use these conditions.

Turn off the UV lamp if machine is not scheduled for use for next two days.

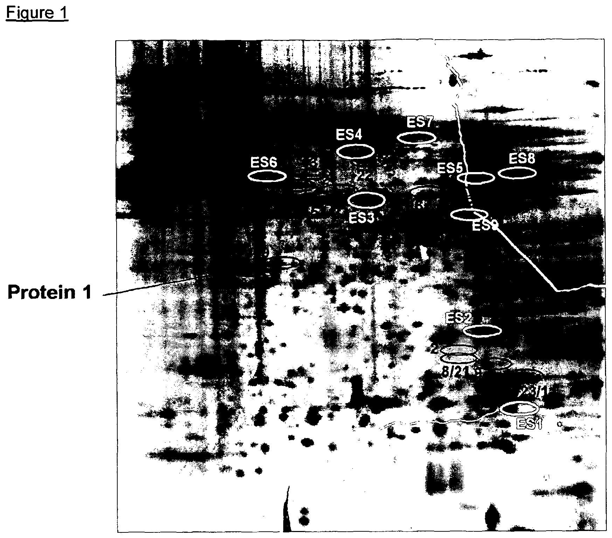

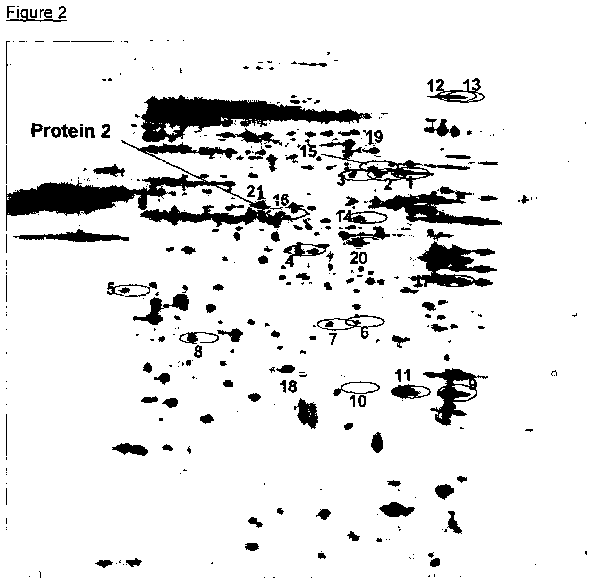

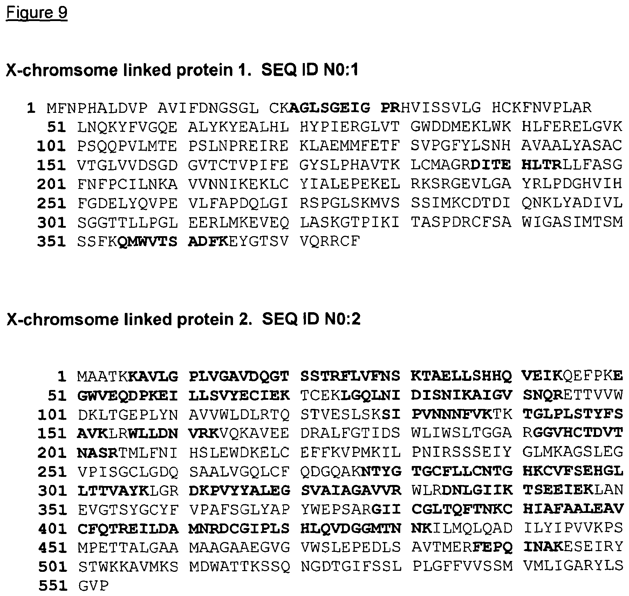

The results of the above examples are shown in Figures 1 and 2, that is gel scanning followed by mass-spectrometry analysis showed the two-dimensional gel electrophoresis position of X-chromosome linked proteins 1 and 2 as shown in Figures 1 and 2. The sequences of Proteins 1 and 2 are given in Figure 9.

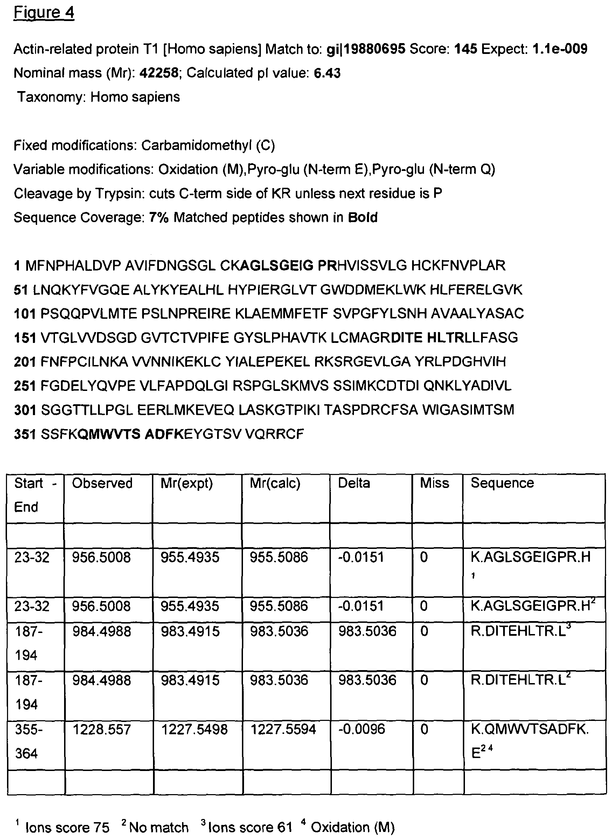

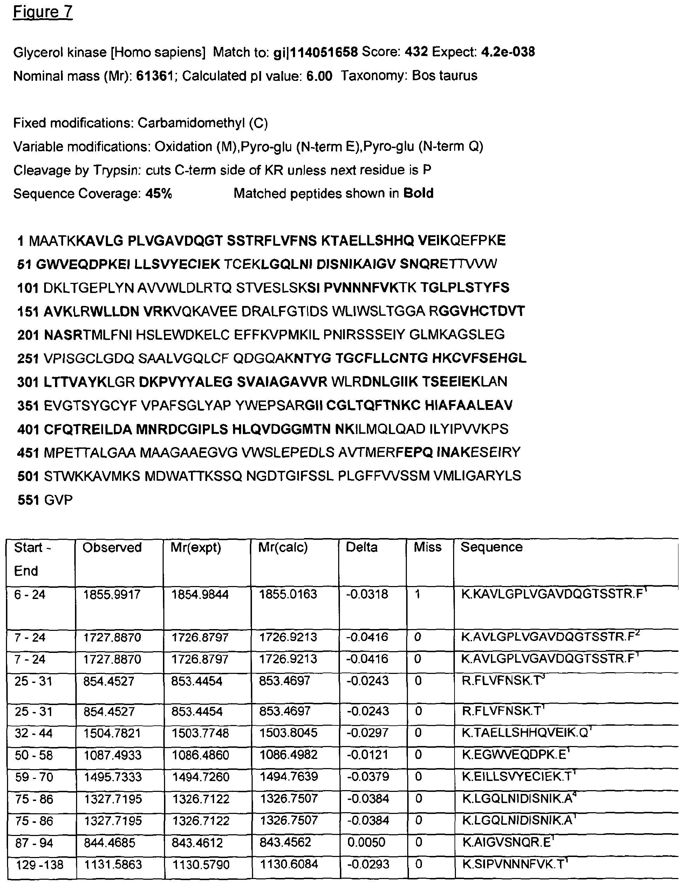

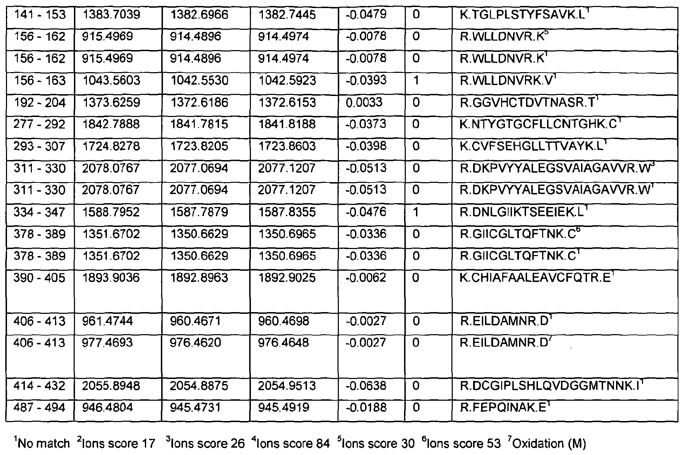

The gel spots for X-chromosome linked proteins 1 and 2 were identified by picking the spots from the gel and subjecting them to mass-spectrometry analysis to establish peptide sequence data. This peptide sequence data was subsequently entered into MASCOT, a search engine that uses mass spectrometry data to identify proteins from primary sequence databases. The results for this are shown in Figures 4 and 7. Neither protein 1 nor protein 2 were previously known to be preferentially expressed by X-chromosome bearing cells, especially when compared to Y-chromosome bearing cells. In fact, protein 2 maps to chromosome 9 (an autosome) in cattle and so its preferential expression by X-chromosome bearing sperm cells is wholly unexpected.

### Cattle. : Antibody Sourcing

### a) Protein 1

The following peptides were selected from Protein 1 according to the description in Heid et al (2002).
1. Peptide hArp-T1-I (FNPHALDVPAVIF), corresponding to N-terminal amino acid positions 2-14 of human protein Arp-T1;
2. Peptide hArp-T1-II (GWDDMEKLWKHLFERELGVKPSQQ), corresponding to sequence positions 81-104 of human Arp-T1;
3. Peptide hArp-T1-III (GLEERLMKEVEQLASKGTP), corresponding to positions 309 327 of human Arp-T1;
4. Peptide hArp-T1-IV (SADFKEYGTSWQR), corresponding to positions 360-373 close to the C-terminus of human Arp-T1;
5. Peptide hArp-T2-I (LEPEKELSRRPEEVLREYKLPDGN), corresponding to positions 225-248 of human Arp-T2.

All five peptides were synthesized by Cambridge Research Biochemicals Limited (Cleveland, UK), and prepared for immunising sheep. Polyclonal antibodies were raised in sheep and prepared for laboratory use by Micropharm Limited (Newcastle Emlyn, UK).

Primary antibody binding to the cell surface was assessed using a commercially sourced secondary fluorescence labelled (Alexa Flour 488) antibody to sheep immunoglobulin.

### b) Protein 2

Anti-GK human antibody was sourced from Tebu-Bio. (Peterborough, UK).

The Anti-GK antibody was raised in mice.

A fluorescently labelled anti-mouse antibody was purchased from Invitrogen (http://www.invitrogen.com/site/us/en/home.html)

Sperm Preparation and antibody binding were carried out as follows:-
Sperm cells were incubated overnight in TEST-Yolk buffer (Ijaz and Hunter 1989). After incubation the cells were centrifuged at 1,000g (-2,800 rpm) for 30 minutes at 5°C. after spinning, the supernatant was poured off and the cells were resuspended in 10ml of the separation buffer before being transferred into a 15ml tube to be recentrifuged at 1,000g (2,500rpm for 15ml tubes) for 10 minutes at 5°C.

After centrifugation, the supernatant was removed and the cells were resuspended in a further 5ml of the separation buffer. This suspension was then centrifuged once more at 1,000g (2,500rpm for 15ml tubes) for 10 minutes at 5°C before the supernatant was removed and the cells were resuspended in 1ml of the separation buffer.

The resulting suspension was counted and aliquoted into batches of 5 x 10⁶ cells for each antibody to be tested. The aliquots were made up to 100µl with phosphate buffered saline (PBS) and 100µl of antibody was added to each tube and mixed by pipetting.

The tube is then incubated at 37 °C for 1 hour before centrifuging the tube at 3,000xg for 10 minutes and carefully removing the supernatant and resuspending the cells in 200µl PBS.

The centrifuging and resuspending step are repeated and the pellet is resuspended in 100µl PBS. 100µl of the secondary antibody is added calculating dilution such that the concentration given is the total dilution in the 200µl solution.

The sample is then incubated at 37°C in the dark (as fluorescently labelled) for 1 hour and centrifuging at 3,000xg for 10 minutes before carefully removing the supernatant and resuspending in 200µl PBS. The centrifuging step is repeated and the pellet resuspended in 500µl PBS to give a concentration of 1 x 10⁷/ml before being mixed up and down well with a pipette.

A 20µl drop of this solution is placed onto a labelled slide and drop of Mowiol/ Dabco or DAKO (http://www.dako.com/) antifade slide mount is added to each slide before being carefully covered with a large coverslip and left to set overnight in a cupboard/drawer wrapped in tin foil.

The next day each slide is sealed with nail varnish and allowed to dry before being observed under a microscope. The fluorescence patterns on the sperm are assessed by epifluorescence UV microscopy (Olympus BH-2) at 492nm wavelength, x100 and x40 magnification.

Results of sperm cell surface binding trials for sex-chromosome specific antibodies.

### Pigs

### MicroPharm polyclonal antibody to Protein 1

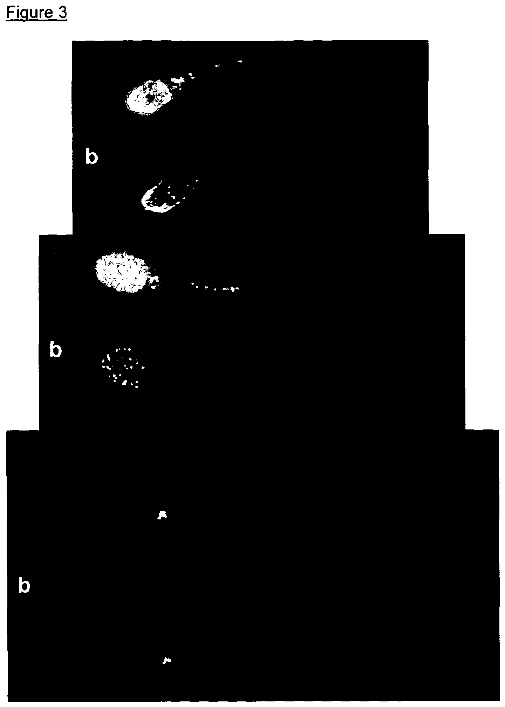

These antibodies were raised in three sheep (denoted 0990, 0991, and 0992) by MicroPharm. Five peptides were synthesised by Cambridge Research Biologicals (CRB) and used to immunise each of the sheep. This included the (single) peptide used to raise the commercial Protein 1 antibody. The final 14 week bleed results are shown below (Figure 3).

With the 14 week antibody surface fluorescence over the entire acrosomal region or apical ridge region of the acrosome was observed. This contrasts with the pre-immune (normal sheep serum), and secondary antibody only controls which did not show the same binding pattern.

This observed pattern was seen with antibodies derived from each of the three sheep, with Sheep 0991 producing the best intensity of signal. See Figure 3 below.

### Commercial Protein 2

The commercial Protein 2 antibody was raised in mice and therefore controls of mouse serum and anti-mouse conjugated secondary antibodies were used.

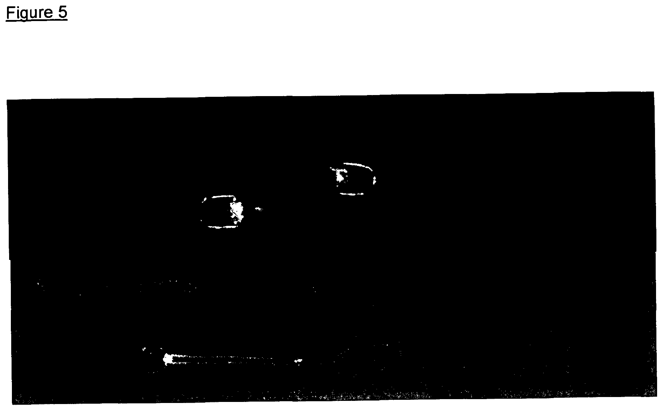

Testing with Protein 2 revealed fluorescence around the apical ridge of the sperm and also some staining of the equatorial subsegment. This clearly shows surface binding (see Figure 5).

### Cattle: MicroPharm polyclonal antibody to Protein 1

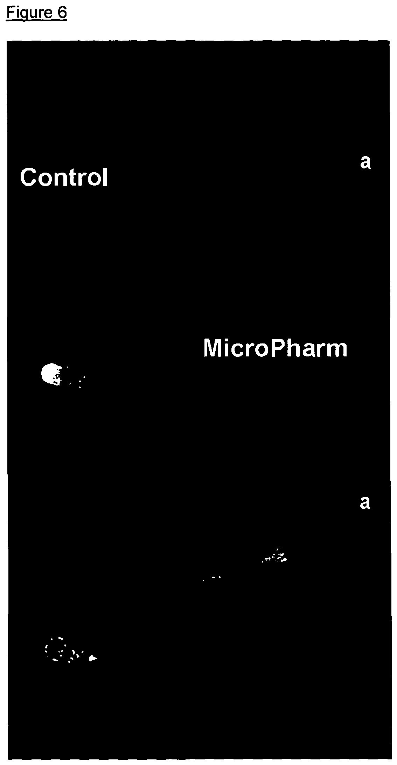

With the MicroPharm antibody to Protein 1, surface fluorescence was observed over the entire acrosomal region or apical ridge region of the acrosome. This contrasts with the normal sheep serum (negative control) which showed no fluorescence (see Figure 6). Secondary antibody only was also used as a control and showed essentially no binding pattern (data not shown).

### Commercial Protein 2

The commercial Protein 2 antibody was raised in mice and therefore controls of mouse serum and anti-mouse conjugated secondary antibodies were used.

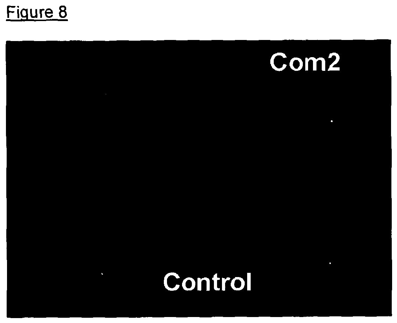

Testing with Protein 2 revealed fluorescence around the apical ridge of the sperm and also some staining of the equatorial subsegment. This clearly shows surface binding (see Figure 8).

### Example 3: Staining of commercially sexed semen for Proteins 1 and Protein 2

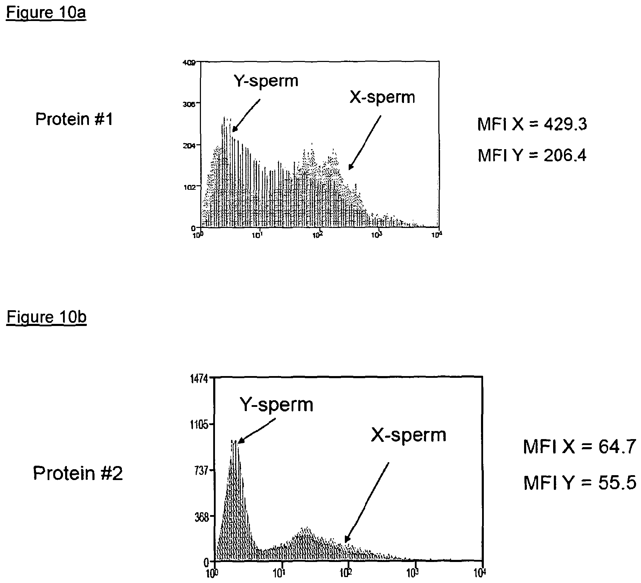

Commercially available samples of sexed semen were stained for the presence of Proteins 1 and Protein 2. Aliquots of 100µl of sperm in PBS (phosphate buffered saline) were labelled with primary antibody at a dilution of 1:150 (in PBS) and incubated for 1 hour at 37°C. Cells were then centrifuged at 3,000 X g for 10 minutes and the supernatant removed. The sperm were resuspended in PBS and the process was repeated 3 times. After this the sperm were again resuspended in PBS and the secondary (fluorescently labelled) antibody was added. This was incubated at 37°C in the dark. Again the sperm were centrifuged at 3,000 X g for 10 minutes and the supernatant removed. The sperm were resuspended in PBS and the process was repeated 3 times. Subsequently the sperm pellet was resuspended in PBS ready for Flow Cytometry analysis. The results are presented in Figure 10, which shows a trace of FACS X and FACS Y-enriched sperm samples incubated in fluorescent antibodies to Proteins 1 and 2 and then cell sorted to measure levels of fluorescence in each population. In more detail, Figure 10 shows sperm numbers are on a linear scale vertically and fluorescence intensity is on a log scale horizontally. In this drawing, fluorescence intensity equates to levels of protein expression on each individual cell in the population of cells.

Looking at Figure 10a, for antibodies to protein 1 if one views the point just before mid-way on the graph where the X and Y plots cross and moves to the right of this a population of X-bearing cells which fluoresce more intensely than Y-bearing cells can be identified. From this data the number of X cells within this population (ie from the point of crossing to the end of the X curve) versus the number of Y-cells from the point of crossing to the end of the Y curve can be estimated. Therefore Figure 10 shows 50% more X than Y, which represents a ratio of 3:2 X:Y which in turn represents an enrichment of 60% X and 40% Y.

Again viewing the point of crossing and moving left, a higher proportion of Y-bearing sperm that fluoresce less than the X-bearing population to the right of the crossing point can be seen. This estimates to about 50% more Y than X. As the same cell numbers have been analysed within each population, then the data is suggestive of the fact that X-bearing cells express Protein 1 greater than Y on average by a factor of 3:2.

Furthermore, the FACS X enriched curve is 90% X and 10% Y, hence it can be deduced that the majority of these 10% Y-bearing cells will be in the lower fluorescing section. Likewise, the Y-enriched cells are 85% Y, and again it can be deduced that the majority of the 15% X bearing cells are in the higher fluorescing section, giving a 60-65% fluorescence in favour of X overall.

This is considered to be conclusive proof of the preferential expression of Protein 1 by the X-bearing sperm cells.

### Example 4: Evidence to show preferential binding of antibodies to Proteins 1 and 2 in X-bearing pig sperm

FACS analysis work with unseparated pig semen incubated with fluorescent antibodies to Proteins 1 and 2 has been performed using a different experimental protocol (see below).

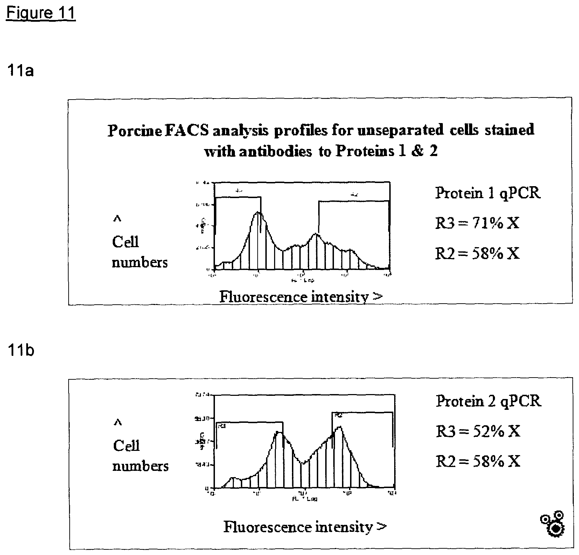

Protein 1 is X-linked in cattle, and is highly likely to be X-linked in the pig (it is X-linked in humans). Protein 2 maps to chromosome 9 in cattle but is also X-linked in humans, and is therefore also highly likely to be X-linked in the pig. Figure 11 shows two FACS separation profiles for unseparated pig sperm incubated in antibodies to Protein 1 and Protein 2.

In this experiment, both FACS analysis profiles (Protein 1 - upper profile, 11a; Protein 2 - lower profile, 11b) appear to show fluorescent antibody binding to unseparated pig cells in a similar pattern - ie, both FACS fluorescence profiles show a dual peak pattern.

In order to complete the analysis, evidence for preferential expression of Proteins 1 and 2 within the least fluorescent cell population in the sample (from left hand peak to left hand tail) and the most fluorescent cell population in the sample (from right hand peak to right hand tail), was sought by collecting cells from these populations and subjecting them to a qPCR test.

qPCR (quantitative polymerase chain reaction), is a molecular test for measuring the relative quantities of X and Y-specific DNA in any given tissue sample. A qPCR technique has been successfully developed by Ovasort at Cardiff for validating X and Y-cell percentages in bovine and porcine sperm samples.

From the above results it can be seen that for antibodies to Protein 1, there is a 71% enrichment for X-bearing cells in the low fluorescence end of the population, and a 58% X-cell enrichment for the cells in the high fluorescence end of the population. For antibodies to Protein 2, there is a 58% X-cell enrichment for cells in the high end of the population only (in this experiment).

This provides conclusive evidence that in pig sperm cells X-linked Protein 1 is preferentially expressed both at lower and higher levels by X-bearing sperm cells compared with Y-bearing cells, and that Protein 2 is preferentially expressed at higher levels by X-bearing sperm cells.

### Example 5

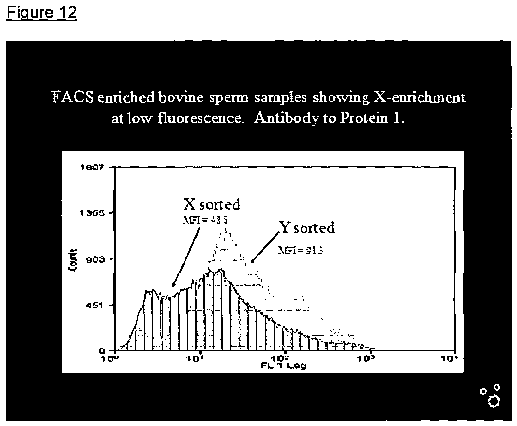

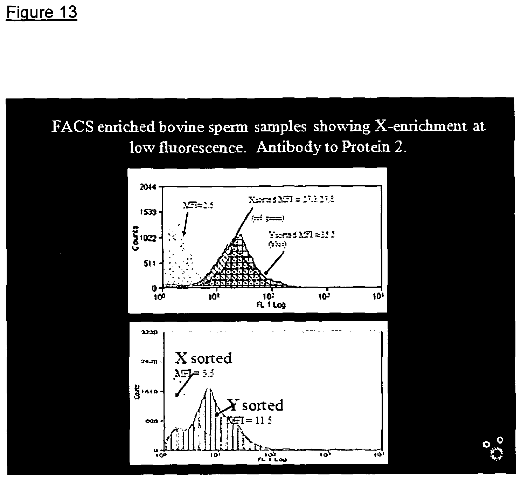

Evidence for preferential expression of Proteins 1 and 2 at low expression rates in cattle. Figure 12 shows that under certain conditions, preferential expression of Protein 1 can occur in bovine sperm cells at low levels of protein expression. The FACS analysis profile of fluorescent antibodies to Protein 1 above clearly shows a unique population of X-bearing cells at the low end of the fluorescence range. This effect has also been shown for Protein 2 in cattle (see Figure 13 and results table below). Figure 13 shows that under certain conditions, preferential expression of Protein 2 can occur in bovine sperm cells at low levels of protein expression.

### Summary

The present inventor's research using bovine sperm has shown X-cell enrichment at each end of an FFE separation range, X - specific antibody binding at the cell surface, and preferential binding of X- specific antibody at the surface of FACS enriched X- bearing cells. The present inventor's research using porcine sperm has shown X - specific antibody binding at the cell surface qPCR validated X-cell enrichment in FACS (surface fluorescence based) separated populations of non-enriched pig sperm cells tagged with X-specific fluorescent antibodies.

A total of 17 experiments made to date show that X-bearing sperm cells exhibit higher fluorescence levels than Y when incubated in fluorescence tagged antibodies to Proteins 1 and 2. These results are summarised in the table below which shows results for FACS analysis of cell surface fluorescence in cattle and pig sperm incubated in antibodies to Proteins 1 and 2.

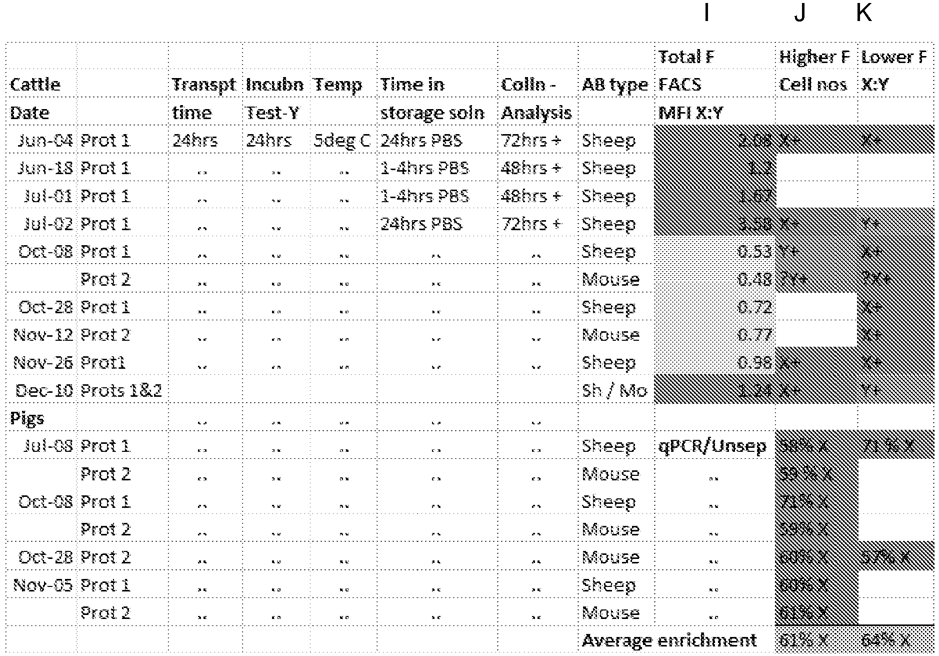

All cattle analyses were performed on FACS X and Y-enriched populations. All pig analyses were performed on unseparated cells from which selected fractions were subjected to qPCR analysis.

Column I = Total F FACS MFI X: Y = ratio of total mean fluorescence index for X enriched: total mean fluorescence index for Y enriched for the sample in question.

Column J (Cattle) = Higher F Cell no.s X: Y = Evidence for either X (X+) or Y (Y+) enrichment at high levels of fluorescence.

Column K (Cattle) = Lower F Cell no.s X: Y = Evidence for either X (X+) or Y (Y+) enrichment at low levels of fluorescence.

Column J and K (Pigs). qPCR data from FACS analysed cell populations at high and low levels of fluorescence.

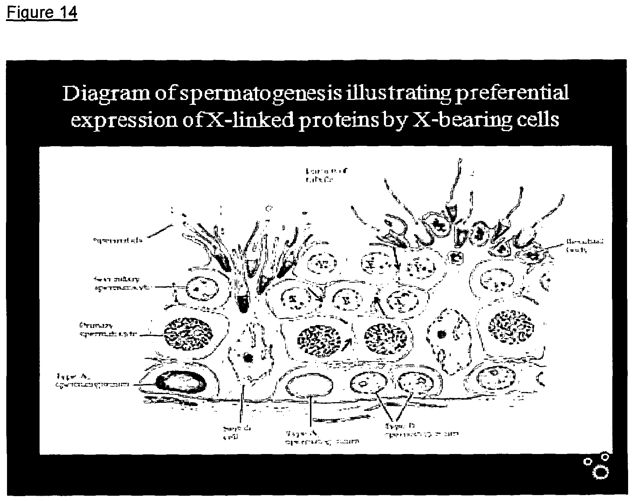

The biological mechanisms behind preferential expression of X-linked Proteins 1 and 2 in pigs and cattle is shown in Figure 14 which is an electron micrograph of the seminiferous epithelium in the mammalian testes. A number of developing spermatocytes have been arbitrarily labelled as X and Y- chromosome bearing cells. These cells develop as a physiological clone and are connected by intercellular bridges (see black arrows), and can therefore share some gene products present in their cytoplasm. X-linked gene products however, can only be produced in X-bearing cells and should therefore, on average, be present at higher levels in X- bearing cells than Y-bearing cells at any one time. The method of the present invention would seek to establish whether a candidate X-specific protein binds to Y-bearing cells and may choose to eliminate any such protein from a sex-selection method according to the degree of any such binding.

There will be occasions where cytoplasmic translational activity in individual cells is extremely high which, in some circumstances, may lead to the preferential translation of minor X-specific gene products in the cytoplasm of Y-bearing cells. However, this is NOT the general rule.

In all cases of X-specific gene transcription, the opportunities for the genetic expression and transcription of an X-specific gene product are 100% in X-bearing cells and 0% in Y-bearing cells, as Y-bearing cells contain no X-specific DNA at all.

### References

Amman, R.P. and Siedel, G.E. (1982). Editors. Prospects for sexing mammalian sperm. Colorado Associated University Press. Boulder, Colorado.
Amman, R.P. (1989). Treatment of sperm to predetermine sex. Theriogenology. 31 (1), 49-60.
Betteridge, K.J. (1984). The folklore of sexing. Theriogenology. 21. 3-6.
Blecher, S.R., Howie, R., Li, S., Detmar, J. and Blahut, L.M. (2000). A new approach to immunological sexing of semen. Theriogenology. 52, 1309-1321.
Cran, D.G. (1992). Gender preselection in mammals. In: Embryonic Development and Manipulation in Animal Production. Trends in Research and Applications. Editors, Lauria, A and Gandolfi, F. Portland Press. London and Chapel Hill. Chapter 10, 125-145.
Cran, D.G. (1993). Approaches to the predetermination of sex in domestic animals. Equine Veterinary Education. 5, 238-241.
Dean, M.D., Good J.M., and Nachman M.W. (2008) Adaptive Evolution of proteins secreted during sperm maturation: An analysis of the mouse epididymal transcriptome. Mol. Biol. Evol. 25(2) 383-392.
Gledhill, B.L. (1983). Control of mammalian sex ratio by sexing sperm. Fertility and Sterility. 40 (3), 572-574.
Gledhill, B.L. (1988). Selection and Separation of X-and Y-Chromosome-Bearing Mammalian Sperm. Gamete Research. 20, 377-395.
Hans W. Heid, Ulrike Figge, Stefanie Winter, Caecilia Kuhn, Ralf Zimbelmann, and Werner W. Franke (2002). Novel Actin-Related Proteins Arp-T1 and Arp-T2 as Components of the Cytoskeletal Calyx of the Mammalian Sperm Head. Experimental Cell Research 279, 177-187.
Holt, W.V. (1982). Functional development of the mammalian sperm plasma membrane. Oxford Reviews of Reproductive Biology. 4, Chapter 10, 195-240.
Ijaz, A. and Hunter, A.G. (1989). Induction of Bovine Sperm Capacitation by TEST-yolk Semen Extender. Journal of Dairy Science. 72, 2683-2690.
Johnson, L.A. (1994). Isolation of X-and Y-bearing sperm for sex preselection. Oxford Reviews of Reproductive Biology. Vol. 16. Oxford Science Publications. Oxford. 303-326.
McEvoy, J.D. (1992). Alteration of the sex ratio. Animal Breeding Abstracts. 60 (2), 97-111.
Oksenberg, D (2008). (WO/2008/028081) Sperm Cell Separation Methods and Compositions Containing Aptamers or Nucleic Acid Sequences for use Therein. International Application No.:PCT/US2007/077300 Publication Date:06.03.2008
Parati, K, Bongioni G, Aleandri R, Galli A (2006) Sex ratio determination in bovine semen: A new approach by quantitative real time PCR. Theriogenology 66 2202-2209.
Parks, J.E., Arion, J.W. and Foote, R.H. (1987). Lipids of Plasma Membrane and Outer Acrosomal Membrane from Bovine Spermatozoa. Biology of Reproduction. 37, 1249-1258.
Vierula, M. and Rajaniemi, H. (1981). Regional Differences in Distribution of Surface Proteins Over the Bull Spermatozoa. International Journal of Andrology. 4(1), 39-48.
Windsor, D.P., Evans, G. and White, I.G. (1993). Sex predetermination by separation of X and Y chromosome bearing sperm: a review. Reproduction, Fertility and Development. 5, 155-171.
Yeh, Y.C., Yang U.C., Huang, S.C. & Lo N.W. (2005). Stage-dependent expression of extra-embryonic tissue-spermatogenesis-homeobox gene 1 (ESX1) protein, a candidate marker for X-chromosome bearing sperm. CSIRO Reproduction, Fertility & Development 17.447-455
Zarutskie, P.W., Muller, C.H., Magone, M., Soules, M.R. (1989). The clinical relevance of sex selection techniques. Fertility and Sterility. 56 (6), 891- 9

## Claims

1. A method of identifying sex-linked proteins in a mammalian sperm cell by identifying selectively expressed X- or Y-chromosome linked proteins at or on the surface of the cell, the method including the steps of:-
i) treating sperm cell samples to remove the accessory proteins and other seminal fluid components;
ii) separating the sperm cell samples into X- and Y-chromosome bearing enriched fractions;
iii) selecting an enriched fraction obtained in step ii) and staining, solublising and subjecting the selected cells to electrophoresis to identify proteins at or on the cell membrane or the cell surface and comparing the results from the selected fraction to those from other fractions to eliminate common proteins;
iv) subjecting the proteins identified in step iii) to mass spectrometry and peptide sequencing; and
v) mapping the sequences from step iv) to recognised databases.

2. A method according to claim 1, **characterised in that** the cells in step i) are capacitated using a capacitating solution or the accessory proteins and other seminal fluid components are removed using a TEST-yolk buffer [an (N-Tris[hydroxymethyl] methyl-2-aminoethane-sulphonic acid)-Tris-yolk buffer] washing step.

3. A method according to claim 1 or claim 2, **characterised in that** the cells in step ii) are separated by free-flow electrophoresis, are, optionally, lysed prior to step iii) and **in that** the electrophoresis of step iii) is selected from the group consisting of a two-dimensional gel electrophoresis, a Sodium Dodecyl Sulphate Polyacrylamide Gel Electrophoresis (SDS-PAGE), and a two-dimensional gel electrophoresis conducted using strips having an immobilised pH gradient.

4. A method according to any one of the preceding claims, **characterised in that** the cells in step iii) are an X-chromosome bearing cell enriched fraction and **in that** the proteins identified in step iii) are X-chromosome specific proteins or proteins whose expression is regulated by the X-chromosome.

5. A method according to any one of the preceding claims, in which the final gel is stained to identify spots of protein.

6. A method according to any one of claims 1 to 4, **characterised in that** the sample of steps iii) and iv) further comprise subjecting the sample to nano-liquid chromatography to produce fractionated protein samples.

7. A method according to claim 6, in which the fractionated protein samples are labelled with an isobaric tagging method prior to mass spectrometry.

8. A method according to any one of the preceding claims, in which the sperm cells in step iii) are treated with dithiothreitol (DTT) to disrupt the DNA therein.

9. A method according to any one of the preceding claims, in which the mammal is a human being, a commercially important, companion, domestic, domesticated or farm animal such as pigs, cattle, sheep, goats, horses, ponies, dogs, cats, or is a captive, zoo or endangered species.

10. Use of the X-chromosome linked protein having the sequence defined in SEQ ID NO: 1 or SEQ ID NO: 2 for the sex selection of mammalian sperm cells.

11. Use of an antibody raised against the X-chromosome linked protein having the sequence defined in SEQ ID NO: 1 or SEQ ID NO: 2 for the sex selection of mammalian sperm cells.

12. Use of antibodies raised against the protein of SEQ ID NO: 1 or SEQ ID NO: 2 for the detection of the presence of said proteins on a mammalian sperm cell.

13. A method of determining the presence or absence of an X-chromosome in a sperm cell by determining the presence or absence of a protein according to claim 10 on a mammalian sperm cell.

14. A method according to claim 13, in which the presence of an X-chromosome in a sperm cell is determined by one of the binding of an antibody according to claim 11 or claim 12 to the mammalian sperm cell, by the binding of greater amounts of antibody according to claim 11 to an X-chromosome bearing mammalian sperm cell, by detecting the overexpression of a protein according to claim 11 in an X-bearing mammalian sperm cell, or by staining with ligands which bind to a protein according to claim 10 and which may be detected visually, where the uptake of ligand is greater in X-bearing cells than in Y-bearing cells.

15. A method according to claim 14 in which the ligand is an aptamer, affibody, antibody or other protein.

## Patentansprüche

1. Verfahren zum Identifizieren geschlechtsgebundener Proteine in einer Säugetier-Samenzelle durch Erkennen selektiv exprimierter X- oder Y-Chromosomen-gebundener Proteine an oder auf der Oberfläche der Zelle, wobei das Verfahren folgende Schritte aufweist:
i) Behandeln von Samenzellenproben, um zusätzliche Proteine und andere Samenflüssigkeitsbestandteile zu entfernen;
ii) Separieren der Samenzellenproben in X- und Y-Chromosom-tragende angereicherte Fraktionen;
iii) Auswählen einer in Schritt ii) erzielten angereicherten Fraktion und Färben, Solubilisieren der ausgewählten Zellen und deren Unterziehen einer Elektrophorese, um Proteine an oder auf der Zellmembran oder der Zelloberfläche zu erkennen und die Ergebnisse der ausgewählten Fraktion mit denen anderer Fraktionen zu vergleichen, um gemeinsame Proteine auszuschließen;
iv) Unterziehen der in Schritt iii) identifizierten Proteine einer Massenspektrometrie und Peptidsequenz; und
v) Zuordnen der Sequenzen aus Schritt iv) zu anerkannten Datenbanken.

2. Verfahren nach Anspruch 1, **dadurch gekennzeichnet, dass** die Zellen in Schritt i) mithilfe einer Kapazitationslösung qualifiziert werden, oder die zusätzlichen Proteine und anderen Samenflüssigkeitsbestandteile werden mithilfe eines Waschschritts mit TEST-Yolk-Puffer [ein (N-Tris[hydroxymethyl]-methyl-2-Aminoethan-Sulfonsäure)-Tris-yolk buffer] entfernt.

3. Verfahren nach Anspruch 1 oder Anspruch 2, **dadurch gekennzeichnet, dass** die Zellen in Schritt ii) durch Free-Flow-Elektrophorese separiert werden, dass sie optional vor Schritt iii) lysiert werden, und **dadurch gekennzeichnet, dass** die Elektrophorese von Schritt iii) ausgewählt ist aus der Gruppe bestehend aus einer zweidimensionalen Gelelektrophorese, einer Natriumdodecylsulfat-Polyacrylamid- Gelelektrophorese (SDS-PAGE) und einer zweidimensionalen Gelelektrophorese, die mithilfe von Streifen mit immobilisiertem pH-Gradienten durchgeführt wird.

4. Verfahren nach einem der vorhergehenden Ansprüche, **dadurch gekennzeichnet, dass** die Zellen in Schritt iii) eine X-Chromosom-tragende angereicherte Zellfraktion sind, und **dadurch gekennzeichnet, dass** die in Schritt iii) identifizierten Proteine X-Chromosom-spezifische Proteine oder Proteine sind, deren Expression durch das X-Chromosom geregelt ist.

5. Verfahren nach einem der vorhergehenden Ansprüche, wobei das finale Gel gefärbt wird, um Proteinspots zu identifizieren.

6. Verfahren nach einem der Ansprüche 1 bis 4, **dadurch gekennzeichnet, dass** die Proben der Schritte iii) und iv) ferner umfassen, dass die Probe einer Nano-Flüssigkeitschromatographie unterzogen wird, um fraktionierte Proteinproben herzustellen.

7. Verfahren nach Anspruch 6, wobei die fraktionierten Proteinproben vor der Massenspektrometrie mit einer Isobarenmarkierungsmethode gekennzeichnet werden.

8. Verfahren nach einem der vorhergehenden Ansprüche, wobei die Samenzellen in Schritt iii) mit Dithiothreitol (DTT) behandelt werden, um die darin enthaltene DNA zu unterbrechen.

9. Verfahren nach einem der vorhergehenden Ansprüche, wobei das Säugetier ein Mensch, ein wirtschaftlich wichtiges Begleit-, Haus-, domestiziertes oder Nutztier, beispielsweise Schweine, Rinder, Schafe, Ziegen, Pferde, Ponys, Hunde, Katzen, oder eine gefangene, im Zoo lebende oder gefährdete Spezies ist.

10. Verwendung des X-Chromosom-verbundenen Proteins mit der in SEQ ID NO: 1 oder SEQ ID NO: 2 definierten Sequenz für die Geschlechtsauswahl der Säugetiersamenzellen.

11. Verwendung eines Antikörpers gegen das X-Chromosom-verbundene Protein mit der in SEQ ID NO: 1 oder SEQ ID NO: 2 definierten Sequenz für die Geschlechtsauswahl der Säugetiersamenzellen.

12. Verwendung von Antikörpern gegen das Protein in SEQ ID NO: 1 oder SEQ ID NO: 2 für den Nachweis der Gegenwart dieser Proteine in einer Säugetiersamenzelle.

13. Verfahren zum Feststellen der Gegenwart oder Abwesenheit eines X-Chromosoms in einer Samenzelle durch Bestimmen der Gegenwart oder Abwesenheit eines Proteins nach Anspruch 10 in einer Säugetiersamenzelle.

14. Verfahren nach Anspruch 13, wobei die Gegenwart eines X-Chromosoms in einer Samenzelle durch eines von Bindung eines Antikörpers nach Anspruch 11 oder Anspruch 12 an die Säugetiersamenzelle, Bindung einer größeren Menge von Antikörpern nach Anspruch 11 an eine X-Chromosom-tragende Säugetiersamenzelle, Nachweis der Überexpression eines Proteins nach Anspruch 11 in einer X-tragenden Säugetiersamenzelle oder Färbung mit Liganden, die an ein Protein nach Anspruch 10 binden, das visuell entdeckt werden kann, wobei die Ligandaufnahme in X-tragenden Zellen größer ist als in Y-tragenden Zellen, festgestellt wird.

15. Verfahren nach Anspruch 14, wobei der Ligand ein Aptamer, Affibody, Antikörper oder anderes Protein ist.

## Revendications

1. Procédé d'identification de protéines liées au sexe dans un spermatozoïde de mammifère en identifiant de manière sélective des protéines liées au chromosome X ou au chromosome Y exprimées sur le spermatozoïde ou à sa surface, le procédé comprenant les étapes consistant à :
i) traiter les échantillons de spermatozoïdes afin d'éliminer les protéines accessoires et d'autres composants du liquide séminal ;
ii) séparer les échantillons de spermatozoïdes en fractions enrichies porteuses du chromosome X et du chromosome Y ;
iii) sélectionner une fraction enrichie obtenue à l'étape ii) et colorer les spermatozoïdes sélectionnés, les solubiliser et les soumettre à une électrophorèse afin d'identifier des protéines sur les spermatozoïdes, sur leur membrane ou à leur surface, et comparer les résultats de la fraction sélectionnée à ceux d'autres fractions afin d'éliminer les protéines communes ;
iv) soumettre les protéines identifiées à l'étape iii) à une spectrométrie de masse et à un séquençage peptidique ; et
v) cartographier les séquences issues de l'étape iv) dans des bases de données reconnues.

2. Procédé selon la revendication 1, **caractérisé en ce que** les spermatozoïdes de l'étape i) sont capacités grâce à une solution de capacitation ou les protéines accessoires et les autres composants du liquide séminal sont éliminés par une étape de lavage par solution tampon de vicellus pour test [une solution tampon de vicellus Tris d'acide N-Tris[hydroxyméthyl]méthyl-2-aminoéthane-sulfonique].

3. Procédé selon la revendication 1 ou 2, **caractérisé en ce que** les spermatozoïdes de l'étape ii) sont séparés par électrophorèse à écoulement libre, sont, de manière facultative, lysés avant l'étape iii) et **en ce que** l'électrophorèse de l'étape iii) est sélectionnée parmi le groupe constitué d'une électrophorèse sur gel bidimensionnelle, une électrophorèse sur gel de polyacrylamide en présence de dodécylsulfate de sodium (SDS-PAGE) et une électrophorèse sur gel bidimensionnelle réalisée en utilisant des bandelettes présentant un gradient de pH immobilisé.

4. Procédé selon l'une quelconque des revendications précédentes, **caractérisé en ce que** les spermatozoïdes de l'étape iii) sont une fraction enrichie de spermatozoïdes porteurs d'un chromosome X et **en ce que** les protéines identifiées à l'étape iii) sont des protéines spécifiques porteuses d'un chromosome X ou des protéines dont l'expression est régulée par le chromosome X.

5. Procédé selon l'une quelconque des revendications précédentes, dans lequel le gel final est coloré afin d'identifier des spots protéiques.

6. Procédé selon l'une quelconque des revendications 1 à 4, **caractérisé en ce que** l'échantillon des étapes iii) et iv) comprend en outre la soumission de l'échantillon à une chromatographie nanoliquide afin de produire des échantillons de protéines fractionnés.

7. Procédé selon la revendication 6, dans lequel les échantillons de protéines fractionnés sont étiquetés par un procédé d'étiquetage isobare avant la spectrométrie de masse.

8. Procédé selon l'une quelconque des revendications précédentes, dans lequel les spermatozoïdes de l'étape iii) sont traités avec du dithiothréitol (DTT) afin de perturber l'ADN qu'il contient.

9. Procédé selon l'une quelconque des revendications précédentes, dans lequel le mammifère est un être humain, un animal de compagnie, domestique, domestiqué ou de ferme, important d'un point de vue commercial, comme des cochons, des bovins, des moutons, des chèvres, des chevaux, des poneys, des chiens, des chats, ou est une espèce vivant en captivité, dans un zoo ou en danger.

10. Utilisation de la protéine liée au chromosome X dont la séquence est définie dans SEQ ID NO: 1 ou SEQ ID NO: 2 pour la sélection du sexe de spermatozoïdes de mammifère.

11. Utilisation d'un anticorps dirigé contre la protéine liée au chromosome X dont la séquence est définie dans SEQ ID NO: 1 ou SEQ ID NO: 2 pour la sélection du sexe de spermatozoïdes de mammifère.

12. Utilisation d'anticorps dirigés contre la protéine de séquence SEQ ID NO: 1 ou SEQ ID NO: 2 pour la détection de la présence desdites protéines sur un spermatozoïde de mammifère.

13. Procédé de détermination de la présence ou de l'absence d'un chromosome X dans un spermatozoïde en déterminant la présence ou l'absence d'une protéine selon la revendication 10 sur un spermatozoïde de mammifère.

14. Procédé selon la revendication 13, dans lequel la présence d'un chromosome X dans un spermatozoïde est déterminée par la liaison d'un anticorps selon la revendication 11 ou selon la revendication 12 au spermatozoïde de mammifère, par la liaison de plus grandes quantités d'anticorps selon la revendication 11 à un spermatozoïde de mammifère porteur d'un chromosome X, par la détection de la surexpression d'une protéine selon la revendication 11 dans un spermatozoïde de mammifère porteur d'un chromosome X, ou par la coloration avec des ligands qui se lient à une protéine selon la revendication 10 et qui peuvent être détectés visuellement, la fixation du ligand étant supérieure dans les spermatozoïdes porteurs du chromosome X que dans les spermatozoïdes porteurs du chromosome Y.

15. Procédé selon la revendication 14, dans lequel le ligand est un aptamère, un afficorps, un anticorps ou une autre protéine.


### SEQUENCE LISTING

<110> Ovasort Ltd
<120> Identification Of Sex-linked Proteins
<130> JMH/8011PCT00
<150> GB0910826.7
   <151> 2009-06-23
<160> 2
<170> PatentIn version 3.3
<210> 1
   <211> 376
   <212> PRT
   <213> Homo sapiens
<400> 1
<210> 2
   <211> 553
   <212> PRT
   <213> Bos taurus
<400> 2
   Met Ala Ala Thr Lys Lys Ala Val Leu Gly Pro Leu Val Gly Ala Val
   1               5                   10                  15
   Asp Gln Gly Thr Ser Ser Thr Arg Phe Leu Val Phe Asn Ser Lys Thr
               20                  25                  30
   Ala Glu Leu Leu Ser His His Gln Val Glu Ile Lys Gln Glu Phe Pro
           35                  40                  45
   Lys Glu Gly Trp Val Glu Gln Asp Pro Lys Glu Ile Leu Leu Ser Val
       50                  55                  60
   Tyr Glu Cys Ile Glu Lys Thr Cys Glu Lys Leu Gly Gln Leu Asn Ile
   65                  70                  75                  80
   Asp Ile Ser Asn Ile Lys Ala Ile Gly Val Ser Asn Gln Arg Glu Thr
                   85                  90                  95
   Thr Val Val Trp Asp Lys Leu Thr Gly Glu Pro Leu Tyr Asn Ala Val
               100                 105                 110
   Val Trp Leu Asp Leu Arg Thr Gln Ser Thr Val Glu Ser Leu Ser Lys
           115                 120                 125
   Ser Ile Pro Val Asn Asn Asn Phe Val Lys Thr Lys Thr Gly Leu Pro
       130                 135                 140
   Leu Ser Thr Tyr Phe Ser Ala Val Lys Leu Arg Trp Leu Leu Asp Asn
   145                 150                 155                 160
   Val Arg Lys Val Gln Lys Ala Val Glu Glu Asp Arg Ala Leu Phe Gly
                   165                 170                 175
   Thr Ile Asp Ser Trp Leu Ile Trp Ser Leu Thr Gly Gly Ala Arg Gly
               180                 185                 190
   Gly Val His Cys Thr Asp Val Thr Asn Ala Ser Arg Thr Met Leu Phe
           195                 200                 205
   Asn Ile His Ser Leu Glu Trp Asp Lys Glu Leu Cys Glu Phe Phe Lys
       210                 215                 220
   Val Pro Met Lys Ile Leu Pro Asn Ile Arg Ser Ser Ser Glu Ile Tyr
   225                 230                 235                 240
   Gly Leu Met Lys Ala Gly Ser Leu Glu Gly Val Pro Ile Ser Gly Cys
                   245                 250                 255
   Leu Gly Asp Gln Ser Ala Ala Leu Val Gly Gln Leu Cys Phe Gln Asp
               260                 265                 270
   Gly Gln Ala Lys Asn Thr Tyr Gly Thr Gly Cys Phe Leu Leu Cys Asn
           275                 280                 285
   Thr Gly His Lys Cys Val Phe Ser Glu His Gly Leu Leu Thr Thr Val
       290                 295                 300
   Ala Tyr Lys Leu Gly Arg Asp Lys Pro Val Tyr Tyr Ala Leu Glu Gly
   305                 310                 315                 320
   Ser Val Ala Ile Ala Gly Ala Val Val Arg Trp Leu Arg Asp Asn Leu
                   325                 330                 335
   Gly Ile Ile Lys Thr Ser Glu Glu Ile Glu Lys Leu Ala Asn Glu Val
               340                 345                 350
   Gly Thr Ser Tyr Gly Cys Tyr Phe Val Pro Ala Phe Ser Gly Leu Tyr
           355                 360                 365
   Ala Pro Tyr Trp Glu Pro Ser Ala Arg Gly Ile Ile Cys Gly Leu Thr
       370                 375                 380
   Gln Phe Thr Asn Lys Cys His Ile Ala Phe Ala Ala Leu Glu Ala Val
   385                 390                 395                 400
   Cys Phe Gln Thr Arg Glu Ile Leu Asp Ala Met Asn Arg Asp Cys Gly
                   405                 410                 415
   Ile Pro Leu Ser His Leu Gln Val Asp Gly Gly Met Thr Asn Asn Lys
               420                 425                 430
   Ile Leu Met Gln Leu Gln Ala Asp Ile Leu Tyr Ile Pro Val Val Lys
           435                 440                 445
   Pro Ser Met Pro Glu Thr Thr Ala Leu Gly Ala Ala Met Ala Ala Gly
       450                 455                 460
   Ala Ala Glu Gly Val Gly Val Trp Ser Leu Glu Pro Glu Asp Leu Ser
   465                 470                 475                 480
   Ala Val Thr Met Glu Arg Phe Glu Pro Gln Ile Asn Ala Lys Glu Ser
                   485                 490                 495
   Glu Ile Arg Tyr Ser Thr Trp Lys Lys Ala Val Met Lys Ser Met Asp
               500                 505                 510
   Trp Ala Thr Thr Lys Ser Ser Gln Asn Gly Asp Thr Gly Ile Phe Ser
           515                 520                 525
   Ser Leu Pro Leu Gly Phe Phe Val Val Ser Ser Met Val Met Leu Ile
       530                 535                 540
   Gly Ala Arg Tyr Leu Ser Gly Val Pro
   545                 550